# EUROPÄISCHE PATENTSCHRIFT

(11) **EP 2 024 465 B1**
(45) Veröffentlichungstag und Bekanntmachung des Hinweises auf die Patenterteilung: **13.01.2016**
(21) Anmeldenummer: 07725406.8
(22) Anmeldetag: 21.05.2007
(51) Int. Cl.: C09K 11/06, C09B 17/00, C09B 19/00, C09B 21/00, C09B 57/00

(54) **NEUE MATERIALIEN FÜR ORGANISCHE ELEKTROLUMINESZENZVORRICHTUNGEN**
NEW MATERIALS FOR ORGANIC ELECTROLUMINESCENT DEVICES
NOUVEAUX MATÉRIAUX POUR DISPOSITIFS ÉLECTROLUMINESCENTS ORGANIQUES

(30) Priorität: 31.05.2006 DE 102006025777
(43) Veröffentlichungstag der Anmeldung: 18.02.2009
(73) Patentinhaber: Merck Patent GmbH, 64293 Darmstadt (DE)
(72) Erfinder: BACH, Ingrid, 65719 Hofheim (DE); BUESING, Arne, 65959 Frankfurt am Main (DE); HEUN, Suanne, 65812 Bad Soden (DE); STOESSEL, Philipp, 60487 Frankfurt/Main (DE); HOLBACH, Michael, 61440 Oberursel (DE); KROEBER, Jonas, 60311 Frankfurt (DE); PARHAM, Amir Hossain, 65929 Frankfurt (DE)
(86) Internationale Anmeldenummer: PCT/EP2007/004499
(87) Internationale Veröffentlichungsnummer: WO 2007/137725

(56) Entgegenhaltungen:
- EP-A1- 1 489 154
- WO-A-2004/093207
- JP-A- 2003 267 976
- JP-A- 2005 154 396
- FIELD J E ET AL: "Bridged Triarylamines: A New Class of Heterohelicenes" JOURNAL OF ORGANIC CHEMISTRY, AMERICAN CHEMICAL SOCIETY. EASTON, US, Bd. 68, 2003, Seiten 6071-6078, XP002425435 ISSN: 0022-3263

## Beschreibung

Organische Halbleiter werden für eine Reihe verschiedenartiger Anwendungen, die im weitesten Sinne der Elektronikindustrie zugerechnet werden können, entwickelt. Der Aufbau organischer Elektrolumineszenzvorrichtungen (OLEDs), in denen diese organischen Halbleiter als funktionelle Materialien eingesetzt werden, ist beispielsweise in US 4539507, US 5151629, EP 0676461 und WO 98/27136 beschrieben.

Eine Entwicklung, die sich in den letzten Jahren abzeichnet, ist der Einsatz metallorganischer Komplexe, die Phosphoreszenz statt Fluoreszenz zeigen (M. A. Baldo et al., Appl. Phys. Lett. 1999, 75, 4-6). Aus quantenmechanischen Gründen ist unter Verwendung metallorganischer Verbindungen als Phosphoreszenzemitter eine bis zu vierfache Energie- und Leistungseffizienz möglich. Ob sich diese Entwicklung durchsetzen wird, hängt davon ab, ob entsprechende Device-Kompositionen gefunden werden, die diese Vorteile (Triplett-Emission = Phosphoreszenz gegenüber Singulett-Emission = Fluoreszenz) auch in den OLEDs umsetzen können.

Generell gibt es bei OLEDs, die Triplettemission zeigen, immer noch erhebliche Probleme. So ist die operative Lebensdauer allgemein noch zu gering, was bislang noch der Einführung von phosphoreszierenden OLEDs in hochwertigen und langlebigen Vorrichtungen entgegensteht. Weiterhin ist die Ladungsbalance in phosphoreszierenden Vorrichtungen mit den Matrixmaterialien gemäß dem Stand der Technik noch nicht ausgeglichen. Dies führt zu höheren Spannungen und in Folge zu geringerer Effizienz und Lebensdauer. Weiterhin weisen viele der Matrixmaterialien gemäß dem Stand der Technik keine ausreichend hohe Löslichkeit auf, so dass sich diese Materialien nicht zur Verarbeitung aus Lösung eignen.

In phosphoreszierenden OLEDs wird als Matrixmaterial häufig 4,4'-Bis-(N-carbazolyl)biphenyl (CBP) verwendet. Die Nachteile sind kurze Lebensdauern der damit hergestellten Devices und hohe Betriebsspannungen, die zu geringen Leistungseffizienzen führen. Außerdem weist CBP eine nicht ausreichend hohe Glasübergangstemperatur auf. Des Weiteren hat sich gezeigt, dass CBP für blau emittierende Elektrolumineszenz-Vorrichtungen ungeeignet ist, was in einer schlechten Effizienz resultiert. Außerdem ist der Aufbau der Devices mit CBP komplex, da zusätzlich eine Lochblockierschicht und eine Elektronentransportschicht verwendet werden müssen.

Verbesserte Triplett-Matrixmaterialien, basierend auf Ketoverbindungen und Phosphinoxidverbindungen, sind in WO 04/093207 und in WO 05/003253 beschrieben. Jedoch ist mit den dort beschriebenen Matrixmaterialien die Ladungsbalance im Device noch nicht zufriedenstellend, da diese Verbindungen aufgrund ihres tiefen HOMOs ausschließlich Elektronen transportieren.

In JP 2005/154396 wird neben weiteren Carbazolderivaten auch 4,4'-Bis(N,N'-carbazolyl)benzophenon als Triplettmatrixmaterial aufgeführt. Diese Verbindungen zeigen Nachteile durch die direkte Konjugation zwischen der Carbazolgruppe und der Carbonyleinheit, was zu Charge-Transfer-Komplexen führt.

Überraschend wurde gefunden, dass bipolare Verbindungen, in denen mindestens ein aromatisches Carbonyl und/oder mindestens ein Phosphinoxid mit mindestens einem Carbazol und/oder mindestens einem substituierten Arylamin verknüpft ist, insbesondere dann Verbesserungen gegenüber dem oben genannten Stand der Technik aufweisen, wenn zwischen diesen Gruppen eine Konjugationsunterbrechung vorliegt. Diese Materialien weisen weiterhin den Vorteil auf, dass sie sehr gut in gängigen organischen Lösemitteln löslich sind. Daher eignen sie sich auch zur Herstellung organischer elektronischer Vorrichtungen aus Lösung. Zusätzlich zeigen die Materialien bei Verarbeitung aus Lösung sehr gute Filmbildungseigenschaften. Diese Materialien und deren Verwendung in organischen elektronischen Vorrichtungen sind daher der Gegenstand der vorliegenden Erfindung.

Gegenstand der Erfindung sind Verbindungen enthaltend mindestens ein Strukturelement gemäß Formel (1), 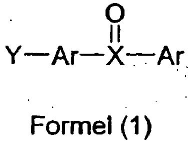 wobei für die verwendeten Symbole und Indizes gilt:
- X: ist bei jedem Auftreten gleich oder verschieden C, P(Ar) oder P(Ar-Y);
- Ar: ist bei jedem Auftreten gleich oder verschieden ein aromatisches oder heteroaromatisches Ringsystem mit 5 bis 60 aromatischen Ringatomen, welches mit einem oder mehreren Resten R¹ substituiert sein kann;
- R¹: ist bei jedem Auftreten gleich oder verschieden H, F, Cl, Br, I, N(Ar¹)₂, CN, NO₂, Si(R²)₃, B(OR²)₂, C(=O)Ar¹, P(=O)(Ar¹)₂, S(=O)Ar¹, S(=O)₂Ar¹, -CR²=CR₂(Ar¹), Tosylat, Triflat, OSO₂R², eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten R² substituiert sein kann, wobei eine oder mehrere nicht benachbarte CH₂-Gruppen durch R²C=CR², C=C , Si(R²)₂, Ge(R²)₂, Sn(R²)₂, C=O, C=S, C=Se, C=NR², P(=O)(R²), SO, SO₂, NR², O, S oder CONR² ersetzt sein können und wobei ein oder mehrere H-Atome durch F, Cl, Br, I, CN oder NO₂ ersetzt sein können, oder ein aromatisches oder heteroaromatisches Ringsystem mit 5 bis 60 aromatischen Ringatomen, das jeweils durch einen oder mehrere Reste R² substituiert sein kann, oder eine Aryloxy-oder Heteroaryloxygruppe mit 5 bis 60 aromatischen Ringatomen, die durch einen oder mehrere Reste R² substituiert sein kann, oder eine Kombination dieser Systeme; dabei können zwei oder mehrere Substituenten R¹ auch miteinander ein mono- oder polycyclisches aliphatisches oder aromatisches Ringsystem bilden;
- Ar¹: ist bei jedem Auftreten gleich oder verschieden ein aromatisches oder heteroaromatisches Ringsystem mit 5 bis 60 aromatischen Ringatomen, welches mit einem oder mehreren Resten R² substituiert sein kann;
- R²: ist bei jedem Auftreten gleich oder verschieden H, F oder ein aliphatischer, aromatischer und/oder heteroaromatischer Kohlenwasserstoffrest mit 1 bis 20 C-Atomen, in dem auch H-Atome durch F ersetzt sein können; dabei können zwei oder mehrere Substituenten R² auch miteinander ein mono- oder polycyclisches aliphatisches oder aromatisches Ringsystem bilden;
- Y: ist bei jedem Auftreten gleich oder verschieden eine Gruppe der Formel (2) oder Formel (3), 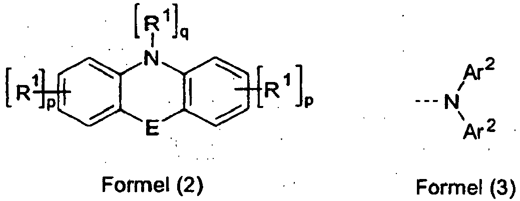 wobei die Einheit gemäß Formel (2) über eine beliebige Position mit Ar verknüpft ist, bevorzugt über N, und die Einheit gemäß Formel (3) über N mit Ar verknüpft ist, wobei R¹ die oben aufgeführte Bedeutung hat und weiterhin gilt:
- E: steht für O, S, N(R¹), P(R¹), P(=O)R¹, C(R¹)₂, Si(R¹)₂ oder für eine Einfachbindung;
- Ar²: ist gleich oder verschieden bei jedem Auftreten eine Aryl- oder Heteroarylgruppe mit 5 bis 20 aromatischen Ringatomen oder eine Triarylamingruppe mit 15 bis 30 aromatischen Ringatomen, welche jeweils mit einem oder mehreren Resten R¹ substituiert sein kann, mit der Maßgabe, dass an mindestens einer Gruppe Ar² mindestens ein Substituent R¹ vorhanden ist, welcher für eine Alkyl- oder für eine Silylgruppe steht;
- p: ist bei jedem Auftreten gleich oder verschieden 0, 1, 2, 3 oder 4;
- q: ist 0 oder 1, wobei q = 0 ist, wenn die Einheit gemäß Formel (2) über den Stickstoff an Ar gebunden ist, und q = 1 ist, wenn die Einheit gemäß Formel (2) über ein anderes Atom als den Stickstoff an Ar gebunden ist;
mit der Maßgabe, dass die Gruppe Ar, welche an X und an Y bindet, nicht durchgängig konjugiert ist, wenn die Verbindung gemäß Formel (1) genau eine Carbonylfunktion aufweist;
ausgenommen ist die folgende Verbindung:

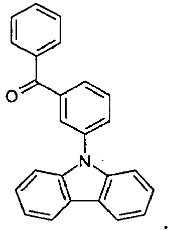

Auch wenn dies aus der oben abgebildeten Struktur hervorgeht, sei hier explizit betont, dass die erfindungsgemäßen Verbindungen auch mehrere Gruppen X=O, also mehrere Carbonyl- und/oder Phosphinoxidgruppen, bzw. auch mehrere Gruppen Y enthalten können.

Bevorzugt sind erfindungsgemäße Verbindungen, welche mindestens zwei Gruppen X=O, also mindestens zwei Carbonyl- und/oder Phosphinoxidgruppen, und/oder mindestens zwei Gruppen Y enthalten.

In einer weiteren bevorzugten Ausführungsform der Erfindung beträgt das Verhältnis von Gruppen X=O zu Gruppen Y zwischen 1:10 und 10:1, besonders bevorzugt zwischen 1 : 5 und 5:1, insbesondere zwischen 1 : 3 und 3:1.

Bevorzugt weisen die erfindungsgemäßen Verbindungen eine Glasübergangstemperatur T_{G} von größer als 70 °C auf, besonders bevorzugt größer als 100 °C, ganz besonders bevorzugt größer als 130 °C.

Eine Arylgruppe im Sinne dieser Erfindung enthält 6 bis 40 C-Atome; eine Heteroarylgruppe im Sinne dieser Erfindung enthält.2 bis 40 C-Atome und mindestens 1 Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzoyl, bzw. ein einfacher heteroaromatischer Cyclus, beispielsweise Pyridin, Pyrimidin, Thiophen, etc., oder eine kondensierte Aryl-oder Heteroarylgruppe, beispielsweise Naphthalin, Anthracen, Phenanthren, Chinolin, Isochinolin, etc., verstanden.

Ein aromatisches Ringsystem im Sinne dieser Erfindung enthält 6 bis 40 C-Atome im Ringsystem. Ein heteroaromatisches Ringsystem im Sinne dieser Erfindung enthält 2 bis 40 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10 % der von H verschiedenen Atome), wie z. B. ein sp³-hybridisiertes C-, N- oder O-Atom, unterbrochen sein können. So sollen beispielsweise auch Systeme wie 9,9'-Spirobifluoren, 9,9-Diarylfluoren, Triarylamin, Diarylether, Stilben, etc. als aromatische Ringsysteme im Sinne dieser Erfindung verstanden werden, ebenso Systeme, in denen zwei oder mehrere Arylgruppen beispielsweise durch eine lineare oder cyclische Alkylgruppe oder durch eine Silylgruppe unterbrochen sind.

Unter einem nicht durchgängig konjugierten aromatischen Ringsystem im Sinne dieser Erfindung wird ein aromatisches Ringsystem, wie oben beschrieben, verstanden, in dem mehrere Aryl- oder Heteroarylgruppen durch eine nicht-konjugierte Einheit, beispielsweise ein sp³-hybridisiertes Kohlenstoffatom, unterbrochen sind. Unter einem nicht durchgängig konjugierten aromatischen Ringsystem, wird weiterhin eine Aryl- oder Heteroarylgruppe verstanden, welche über eine ungerade Anzahl von Kohlenstoffatomen verknüpft ist, wie beispielsweise meta-Phenylen oder 2,6-verknüpftes Pyridin, oder ein aromatisches oder heteroaromatisches Ringsystem, welches mindestens eine derartige Aryl- oder Heteroarylgruppe enthält, da über derartige Positionen keine durchgängige Konjugation möglich ist.

Im Rahmen der vorliegenden Erfindung werden unter einer C₁- bis C₄₀-Alkylgruppe, in der auch einzelne H-Atome oder CH₂-Gruppen durch die oben genannten Gruppen substituiert sein können, besonders bevorzugt die Reste Methyl, Ethyl, n-Propyl, i-Propyl, n-Butyl, i-Butyl, s-Butyl, t-Butyl, 2-Methylbutyl; n-Pentyl, s-Pentyl, Cyclopentyl, n-Hexyl, Cyclohexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl oder Octinyl verstanden. Unter einer C₁- bis C₄₀-Alkoxygruppe werden besonders bevorzugt Methoxy, Trifluormethoxy, Ethoxy, n-Propoxy, i-Propoxy, n-Butoxy, i-Butoxy, s-Butoxy, t-Butoxy oder 2-Methylbutoxy verstanden. Unter einem aromatischen oder heteroaromatischen Ringsystem, mit 5 - 40 aromatischen Ringatomen, welches noch jeweils mit den oben genannten Resten R substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Phenanthren, Pyren, Chrysen, Perylen, Fluoranthen, Naphthacen, Pentacen, Benzpyren, Biphenyl, Biphenylen, Terphenyl, Terphenylen, Fluoren, Spirobifluoren, Dihydrophenanthren, Dihydropyren, Tetrahydropyren, cis- oder trans-Indenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen-, Pyrazin, Phenazin, Phenoxazin, Phenothiazin, Fluorubin, Naphthyridin, Azacarbazol, Benzocarbolin, Phenanthrolin, 1,2,3-Triazol, 1 ;2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Bevorzugte Strukturen, welche mindestens ein Strukturelement gemäß Formel (1) enthalten, sind Verbindungen gemäß den Formeln (4), (5), (6), (7), (8), (9) und (10), 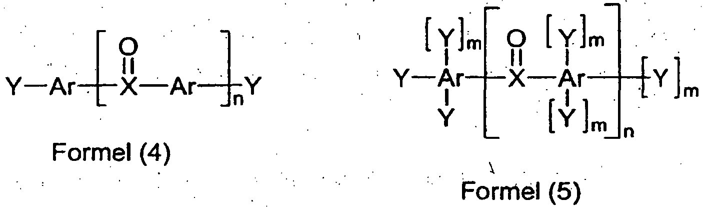 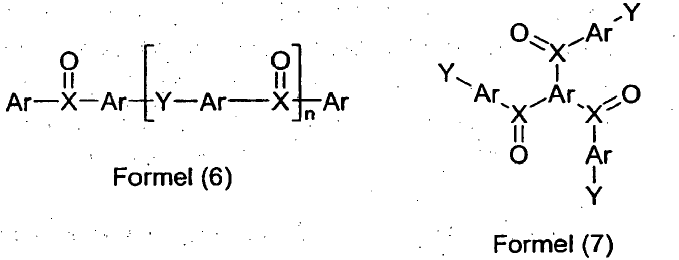 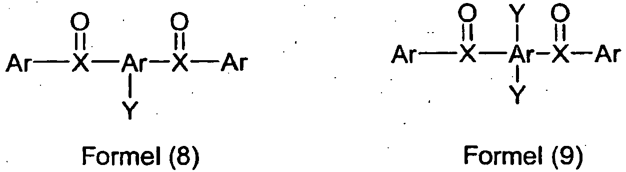 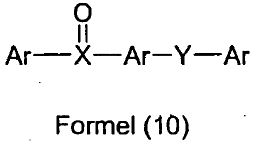 wobei die Symbole und Indizes die oben aufgeführten Bedeutungen haben, und weiterhin gilt:
- m: ist bei jedem Auftreten gleich oder verschieden 0 oder 1;
- n: ist bei jedem Auftreten gleich oder verschieden 1, 2, 3, 4 oder 5.

Bevorzugt sind Verbindungen, welche mindestens ein Strukturelement gemäß Formel (1) enthalten, bzw. Verbindungen gemäß den Formeln (4) bis (10), in denen die Gruppe Ar, welche gleichzeitig an X und an Y bindet, nicht durchgängig konjugiert ist, in denen also Ar eine Konjugationsunterbrechung aufgrund einer nicht-konjugierten Einheit oder einer nicht durchgängig konjugierten Aryleneinheit, beispielsweise einer meta-verknüpften Arylen-Einheit, aufweist, so dass X und Y nicht miteinander in Konjugation stehen.

Bevorzugte Gruppen Ar in Formel (1) bzw. Formel (4) bis (10) enthalten nur Phenyl- und/oder Naphthylgruppen, jedoch keine größeren kondensierten aromatischen Systeme. Daher sind bevorzugte Gruppen Ar aromatische Ringsysteme, welche aufgebaut sind aus Phenyl- und/oder Naphthylgruppen oder Verknüpfungen dieser Systeme, wie beispielsweise Biphenyl, Fluoren, Spirobifluoren, etc.

Besonders bevorzugte Gruppen Ar, welche an X und an Y gebunden sind, sind gewählt aus den Einheiten gemäß Formel (11) bis Formel (17), 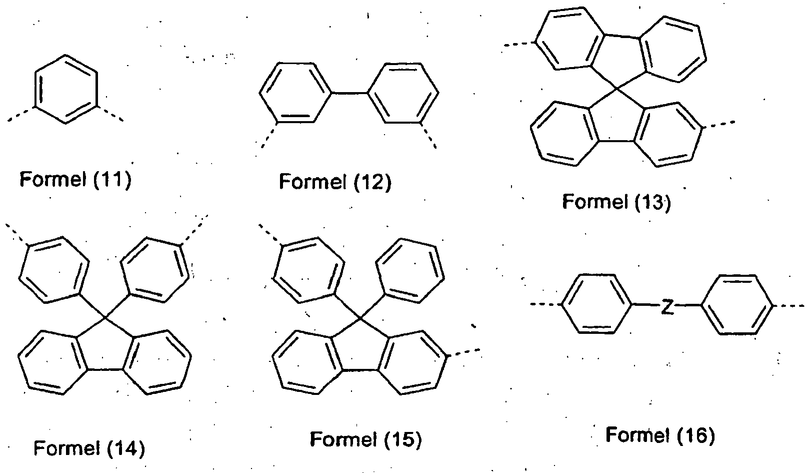 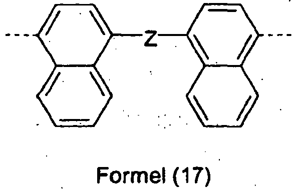 wobei die gestrichelte Bindung jeweils die Verknüpfung mit X und mit Y andeutet, wobei die Einheiten jeweils durch einen oder mehrere Reste R¹ substituiert sein können und weiterhin gilt:
- Z: ist bei jedem Auftreten gleich oder verschieden -[C(R¹)₂]k⁻ , Si(R¹)₂, O oder S;
- k: ist 1, 2, 3, 4, 5 oder 6.

In den Formeln (11) bis (17) ist jeweils nur eine Bindung an X und an Y eingezeichnet. Wenn die erfindungsgemäße Verbindung mehrere Einheiten X=O bzw. mehrere Einheiten Y enthält, wie beispielsweise die Verbindungen gemäß der oben abgebildeten Formel (5), dann weisen die Einheiten gemäß den Formeln (11) bis (17) selbstverständlich auch entsprechend mehr Bindungen zu X bzw. Y auf.

Bevorzugte Einheiten gemäß Formel (2) bzw. Formel (3) sind die folgenden Formeln (2a) bzw. (3a), 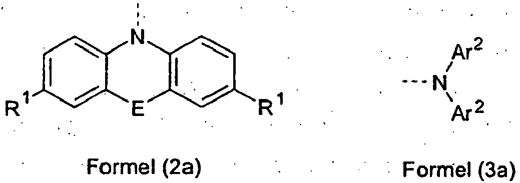 wobei die Einheiten über den Stickstoff mit Ar verknüpft sind, wobei R¹ die oben aufgeführte Bedeutung hat und weiterhin gilt:
- E: steht für eine Einfachbindung, O, S oder N(R¹);
- Ar²: ist gleich oder verschieden bei jedem Auftreten eine Aryl- oder Heteroarylgruppe mit 5 bis 10 aromatischen Ringatomen oder eine Triarylamingruppe mit 18 bis 24 aromatischen Ringatomen, welche jeweils mit einem oder mehreren Resten R¹ substituiert sein kann, mit der Maßgabe, dass an mindestens einer Gruppe Ar² mindestens ein Substituent R¹ vorhanden ist, welcher für eine Alkyl- oder für eine Silylgruppe steht.

Besonders bevorzugt steht Ar² gleich oder verschieden für Phenyl, 1-Naphthyl, 2-Naphthyl, Triphenylamin, Naphthyldiphenylamin oder Dinaphthylphenylamin, wobei mindestens eine dieser Gruppen mit mindestens einer Methyl- oder tert-Butylgruppe oder mindestens einer Gruppe Si(R³)₃ substituiert ist, worin R³ eine Alkylgruppe mit 1 bis 4 C-Atomen darstellt.

Bevorzugt sind weiterhin Verbindungen, enthaltend mindestens ein Strukturelement gemäß Formel (1), bzw. Verbindungen gemäß den Formeln (4) bis (10), in denen das Symbol R¹ gleich oder verschieden bei jedem Auftreten für H, F, Br, N(Ar¹)₂, P(=O)(Ar¹)₂, C(=O)Ar¹, CR²=CR²Ar¹, eine geradkettige Alkylgruppe mit 1 bis 5 C-Atomen oder eine verzweigte Alkylgruppe mit 3 bis 5 C-Atomen, wobei jeweils eine oder mehrere nicht benachbarte CH₂-Gruppen durch -R²C=CR²- oder -O- ersetzt sein können und wobei ein oder mehrere H-Atome durch F ersetzt sein können, oder eine Arylgruppe mit 6 bis 16 C-Atomen oder Heteroarylgruppe mit 2 bis 16 C-Atomen oder eine Spirobifluorengruppe, die jeweils mit einem oder mehreren Resten R² substituiert sein kann, oder eine Kombination aus zwei oder drei dieser Systeme steht. Besonders bevorzugte Reste R¹ sind gleich oder verschieden bei jedem Auftreten H, F, Br, Methyl, Ethyl, iso-Propyl, tert-Butyl, wobei jeweils ein oder mehrere H-Atome durch F ersetzt sein können, oder eine Phenyl-, Naphthyl- oder Spirobifluorenylgruppe, die jeweils mit einem oder mehreren Resten R² substituiert sein kann, oder eine Kombination aus zwei dieser Systeme. Beim Einbau in Polymere, Oligomere oder Dendrimere und bei Verbindungen, die aus Lösung verarbeitet werden, sind auch lineare oder verzweigte Alkylketten mit bis zu 10 C-Atomen bevorzugt. Brom, Boronsäure bzw. Boronsäurederivate als Substituenten sind vor allem für die Verwendung dieser Verbindung zur Herstellung anderer erfindungsgemäßer Verbindungen oder zur Verwendung als Monomer zur Herstellung von Polymeren bevorzugt.

Weiterhin bevorzugt sind symmetrische und symmetrisch substituierte Verbindungen, also Verbindungen, in denen alle Symbole X gleich sind, und Verbindungen, in denen alle Symbole Y gleich sind. Weiterhin bevorzugt sind die Verbindungen symmetrisch bezüglich der Gruppen Ar aufgebaut. Nochmals weiterhin bevorzugt sind in den Strukturen die Substituenten R¹ gleich gewählt.

Beispiele für bevorzugte Verbindungen enthaltend Strukturelemente gemäß Formel (1) sind die im Folgenden abgebildeten Strukturen (1) bis (140). Diese Strukturen können auch durch R¹ substituiert sein. Diese möglichen Substituenten sind in den meisten Fällen der Übersichtlichkeit halber nicht abgebildet.

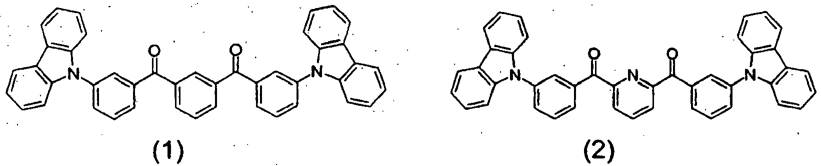

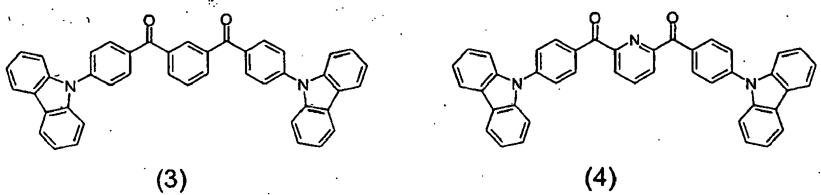

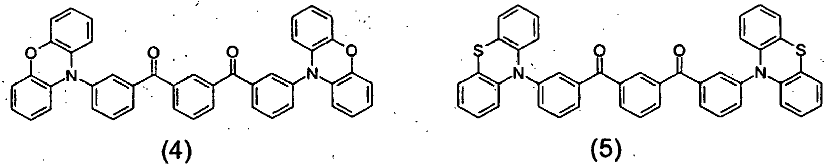

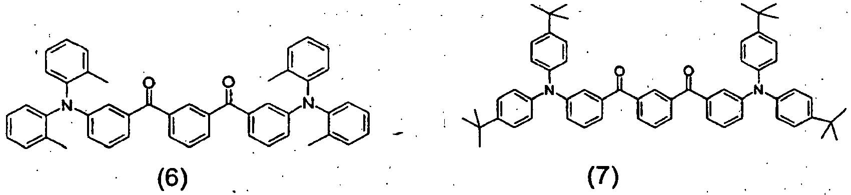

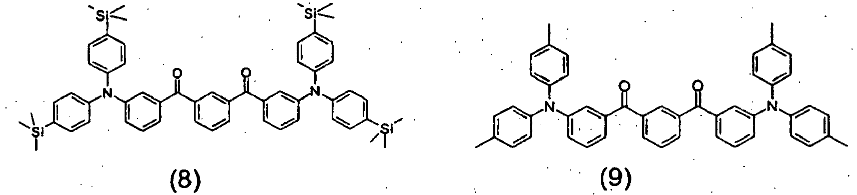

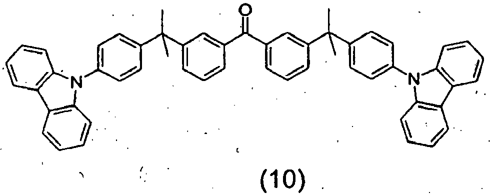

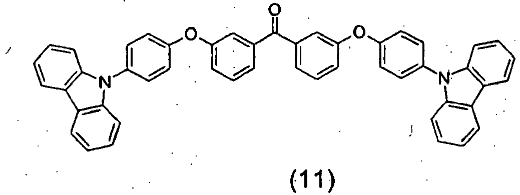

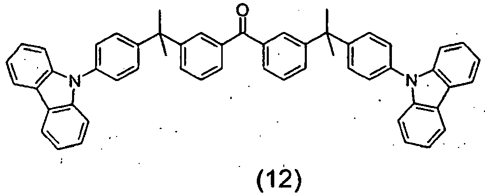

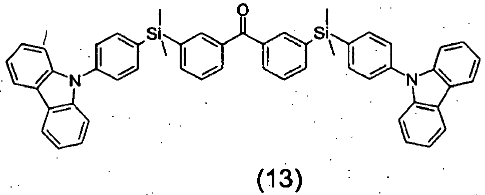

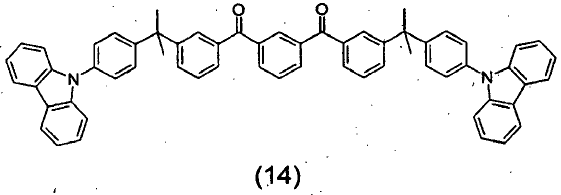

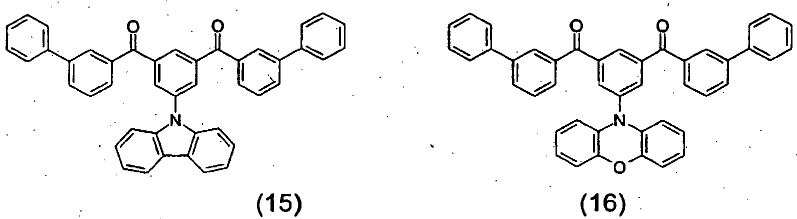

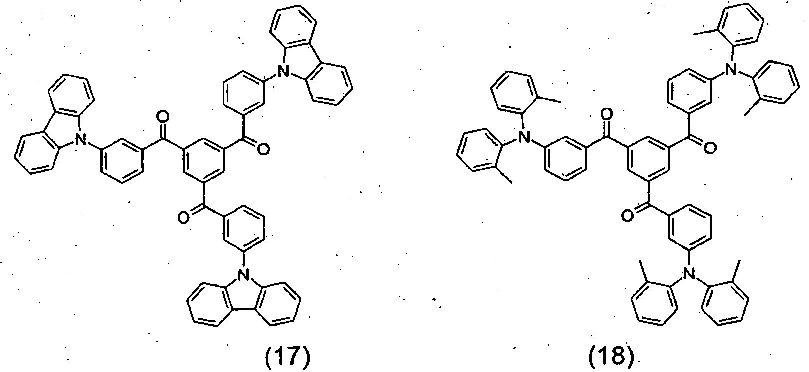

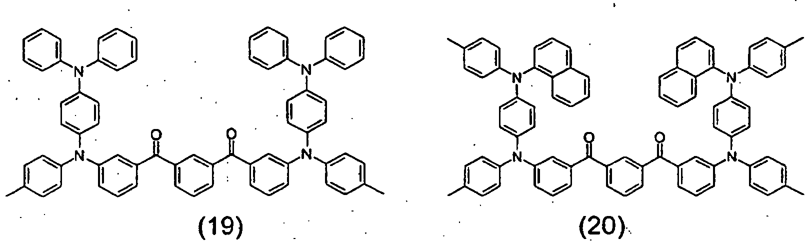

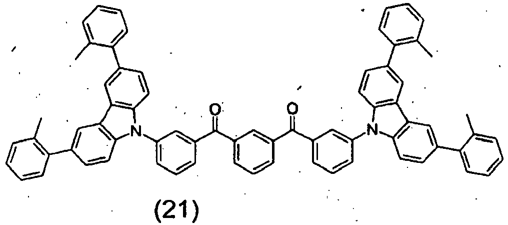

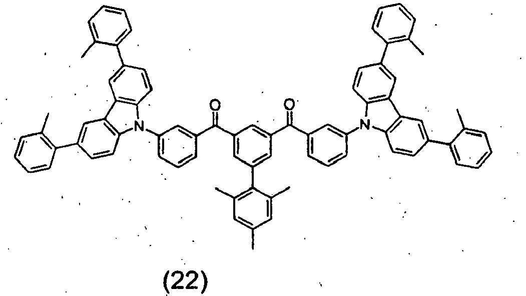

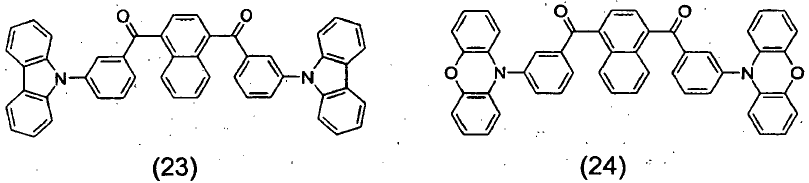

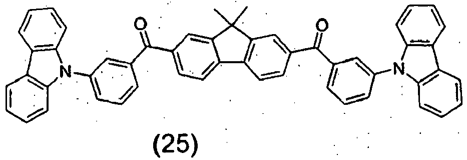

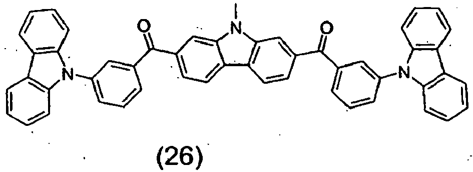

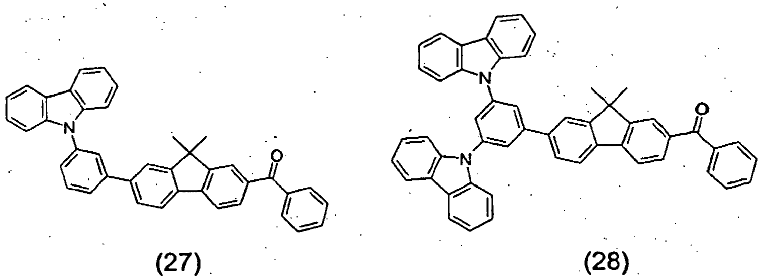

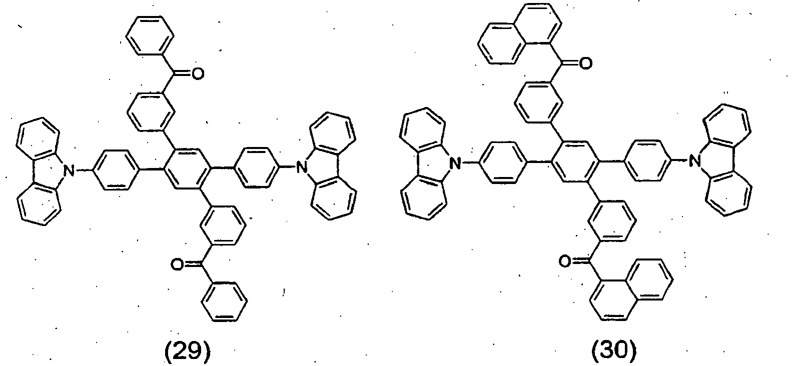

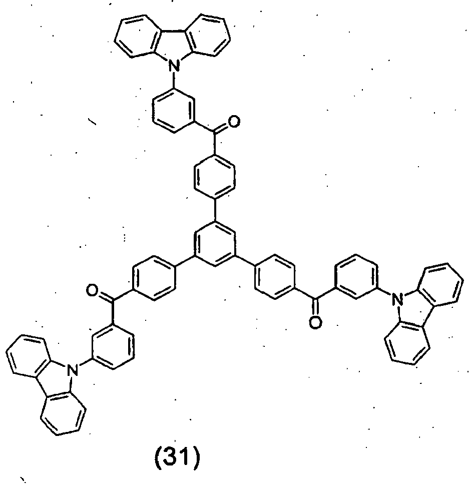

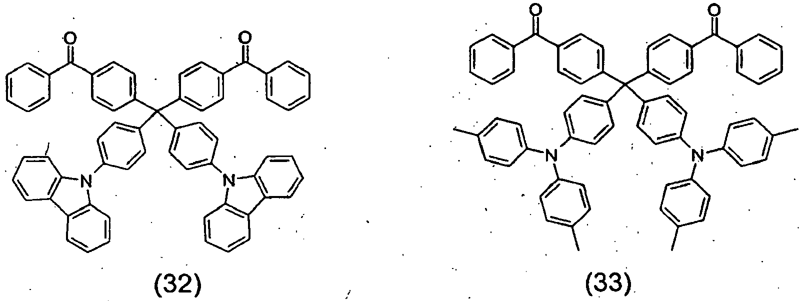

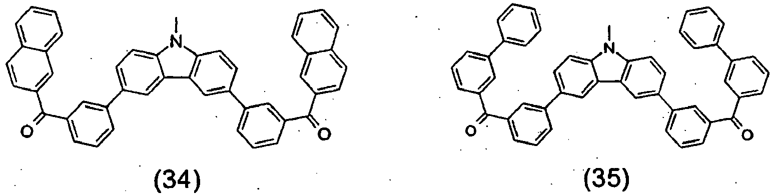

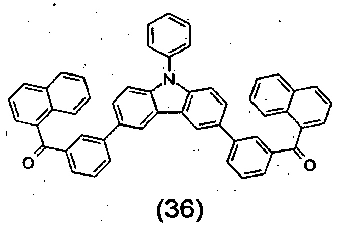

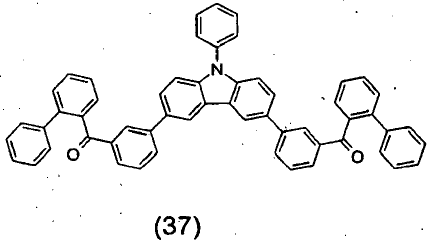

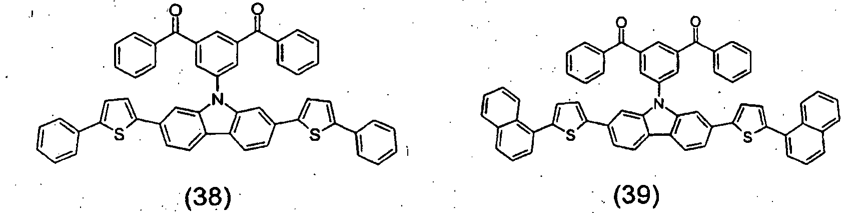

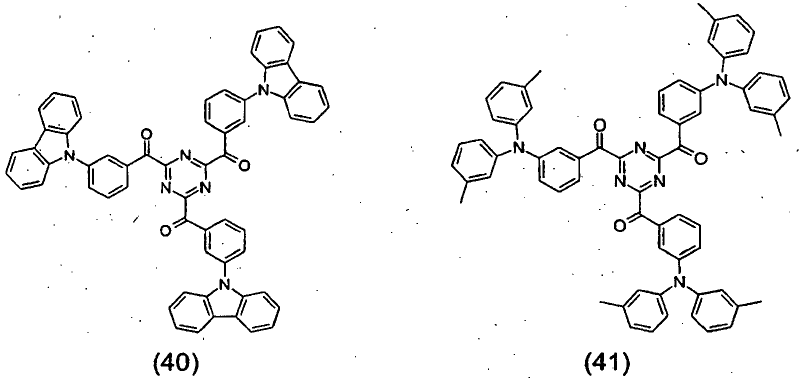

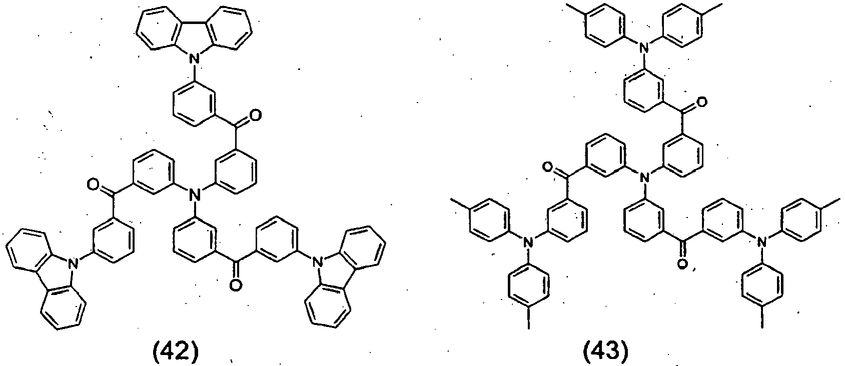

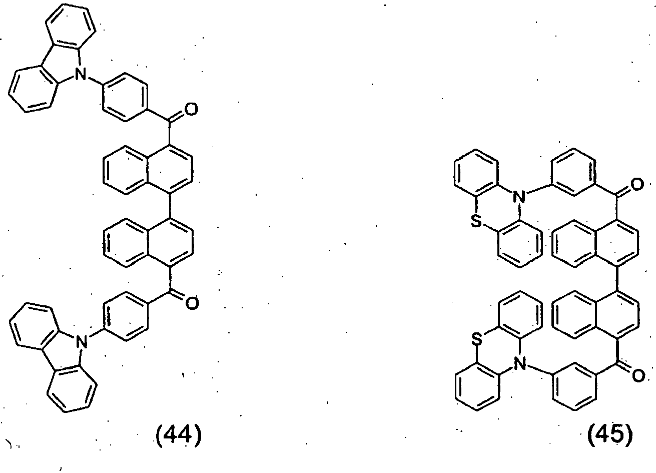

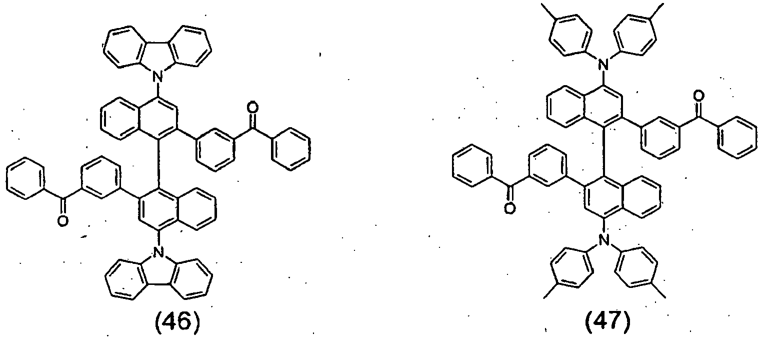

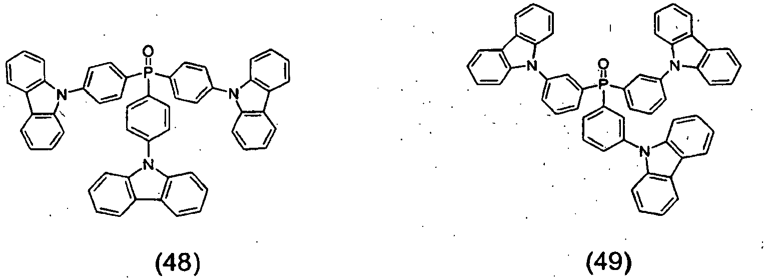

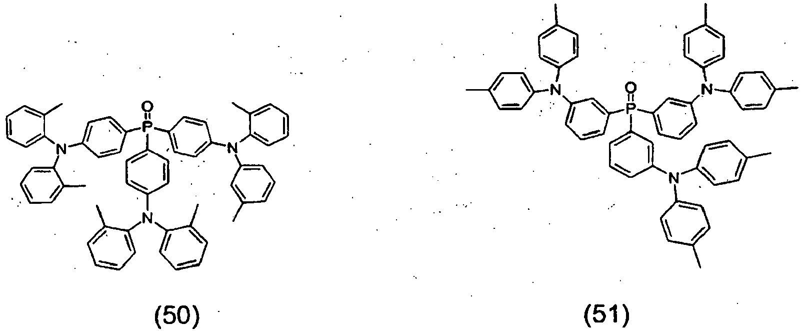

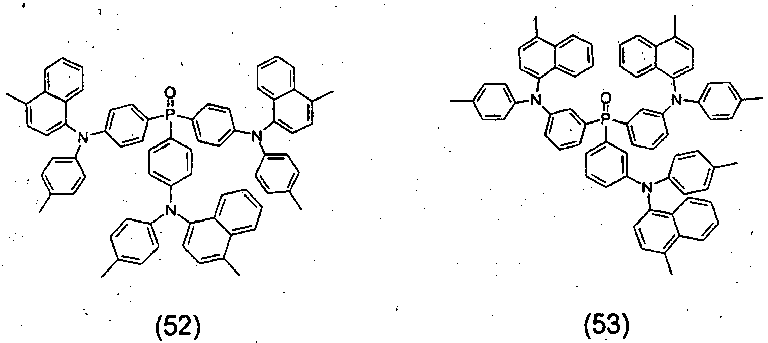

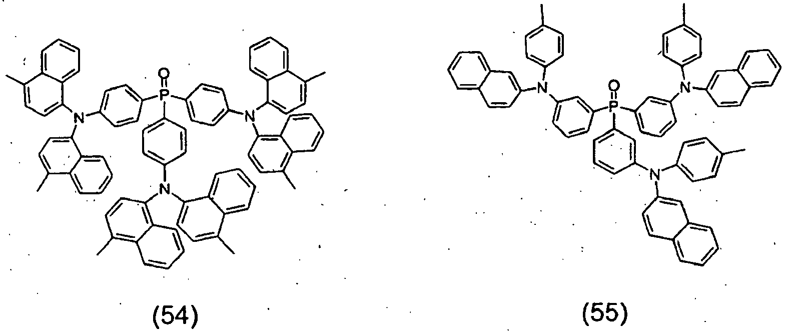

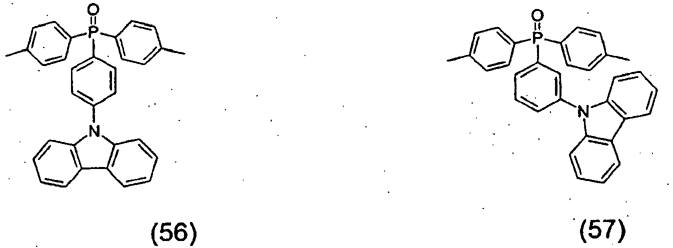

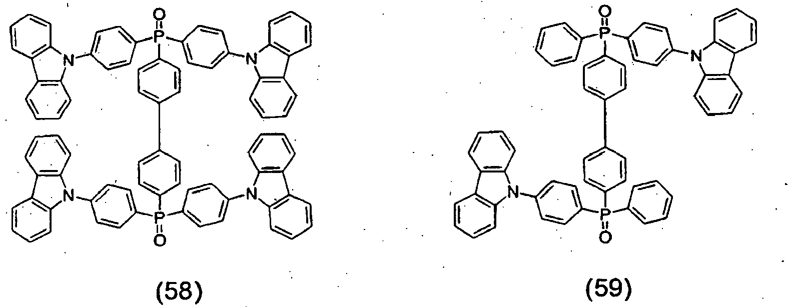

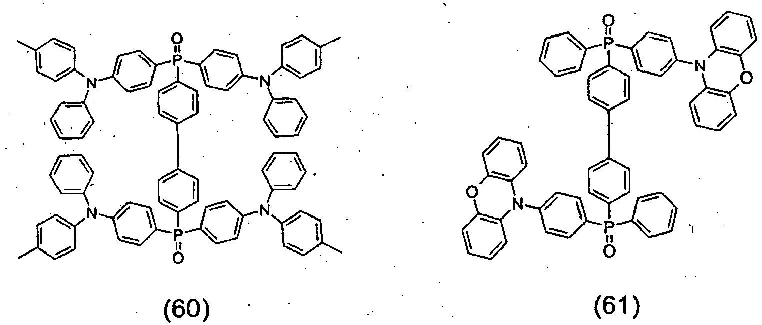

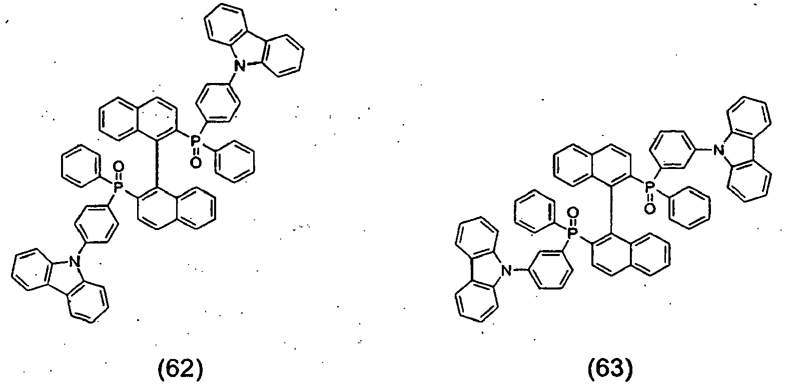

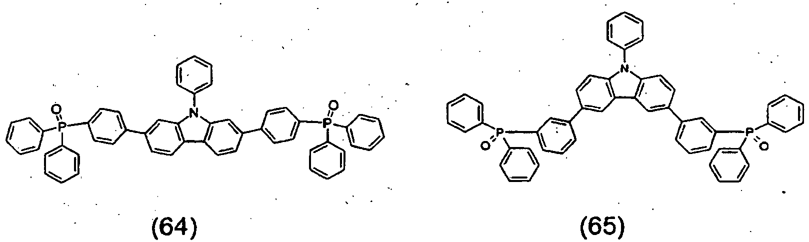

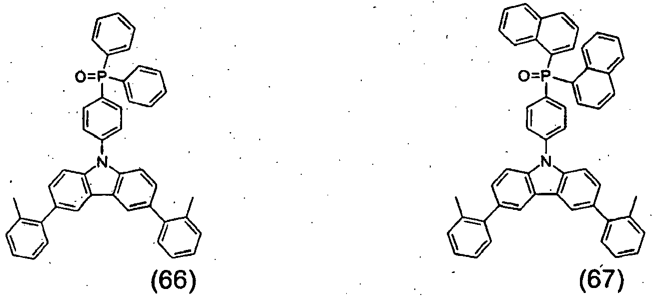

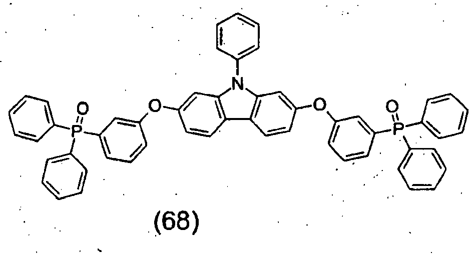

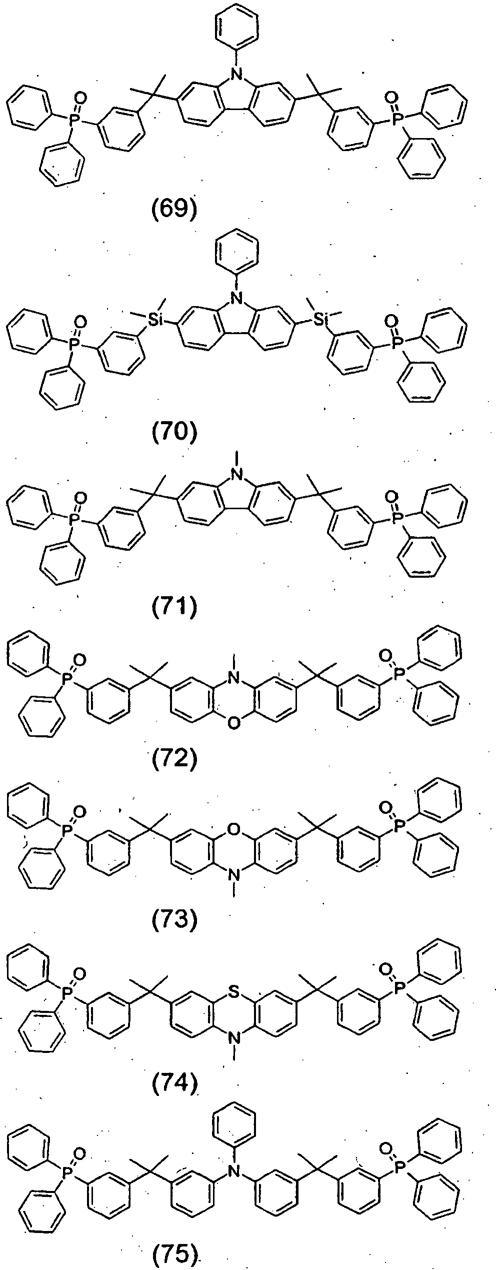

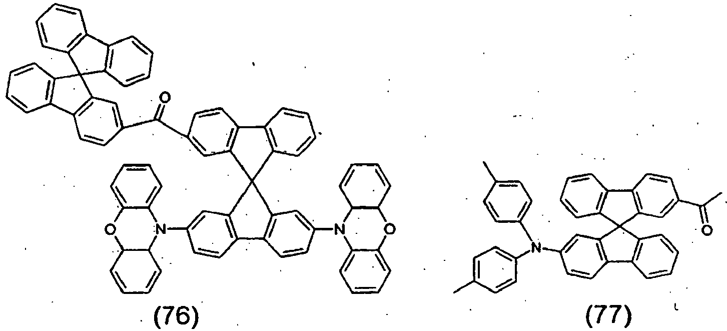

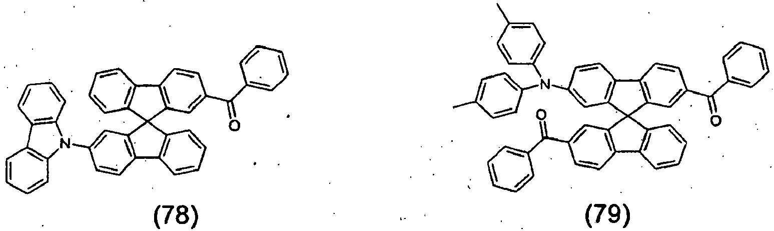

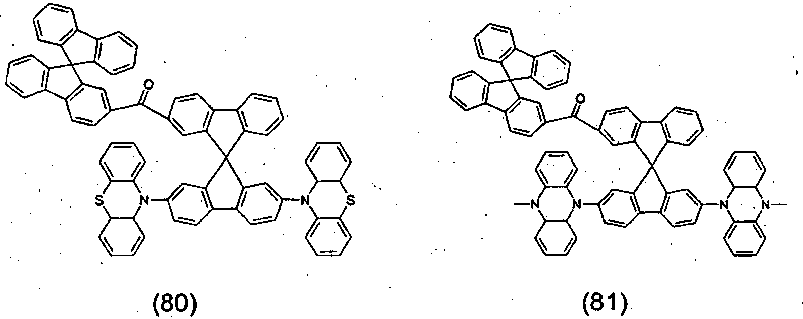

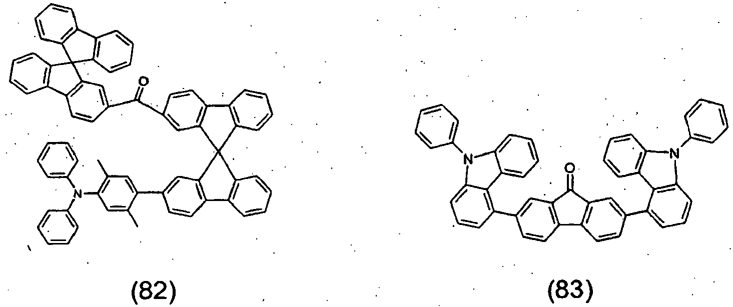

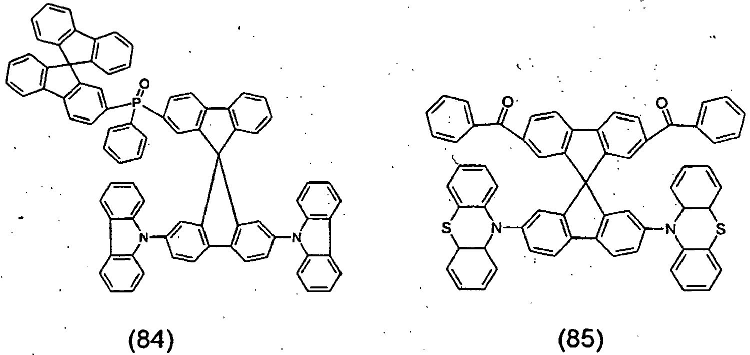

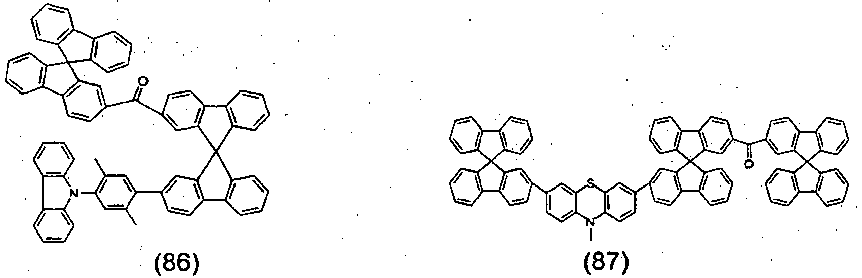

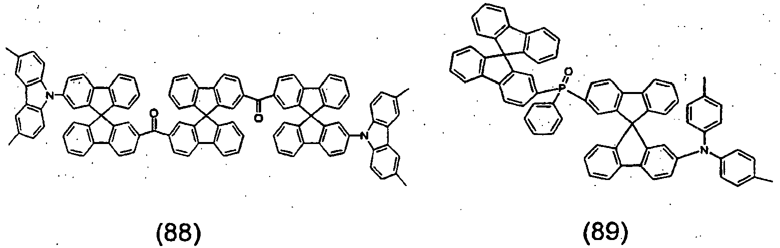

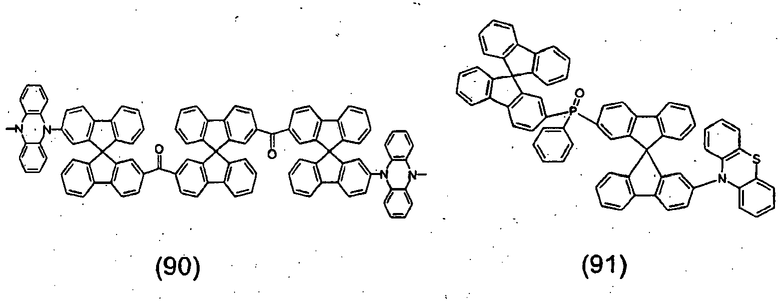

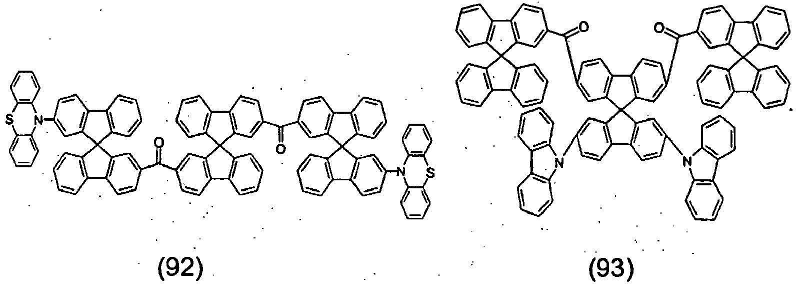

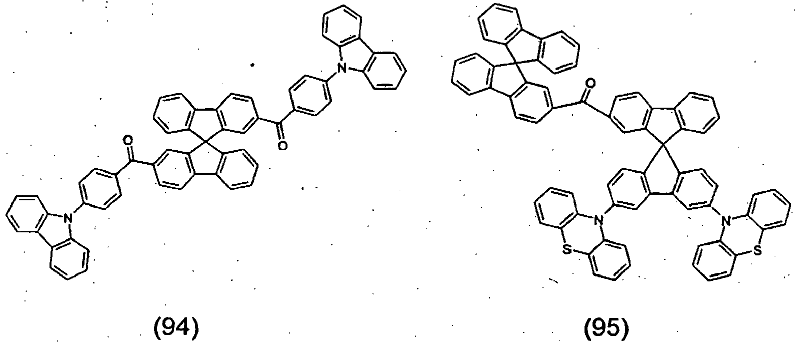

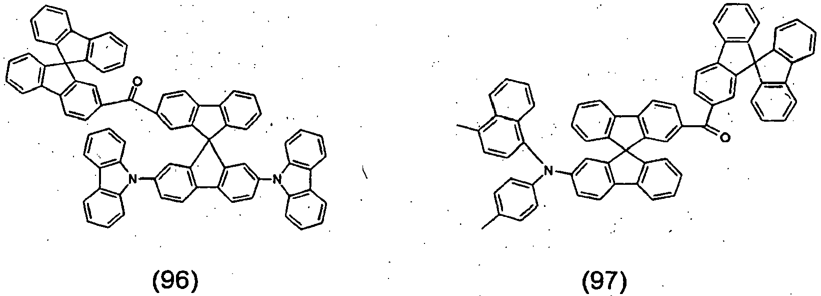

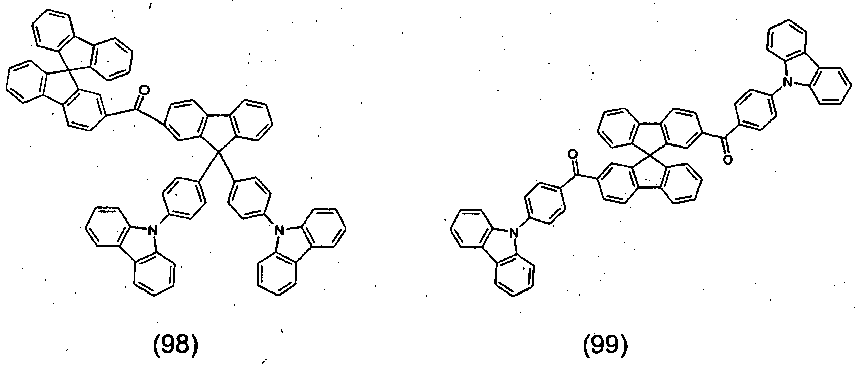

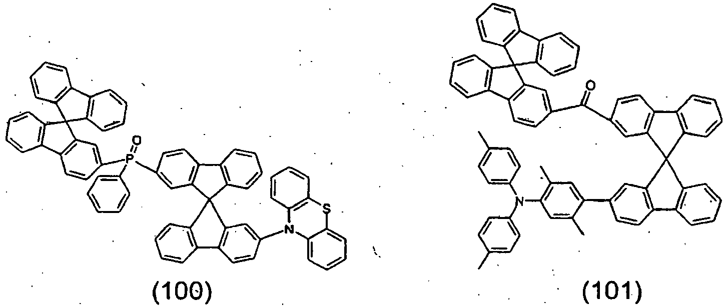

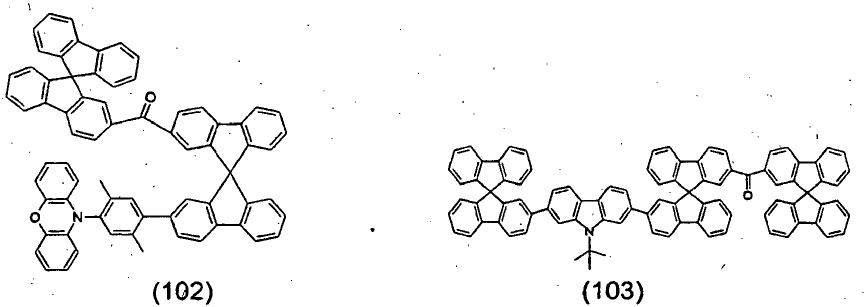

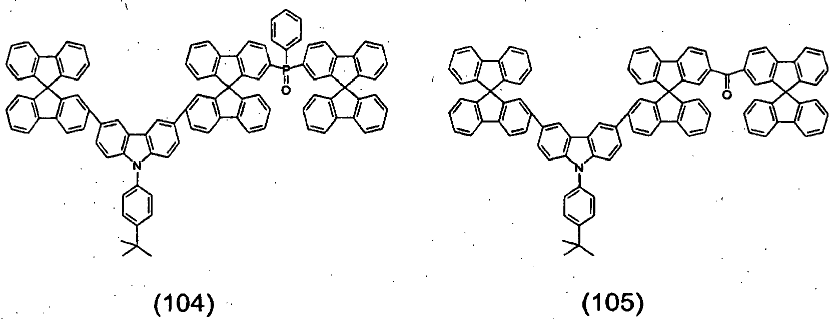

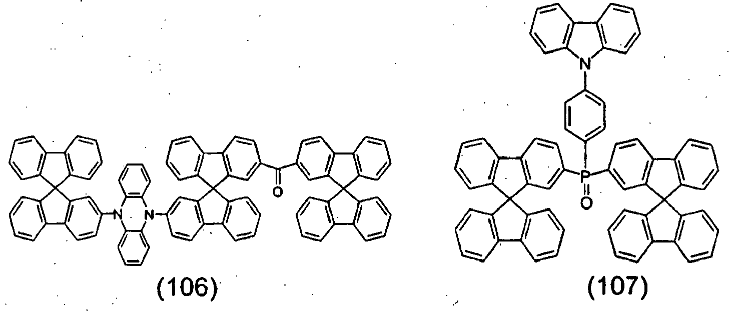

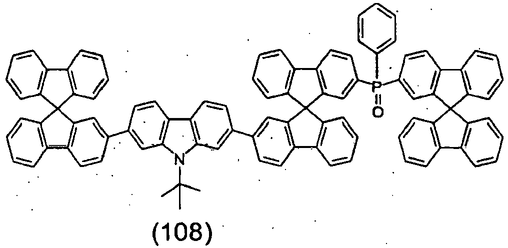

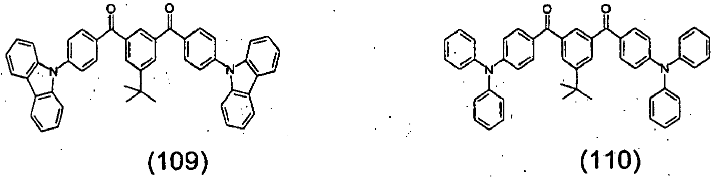

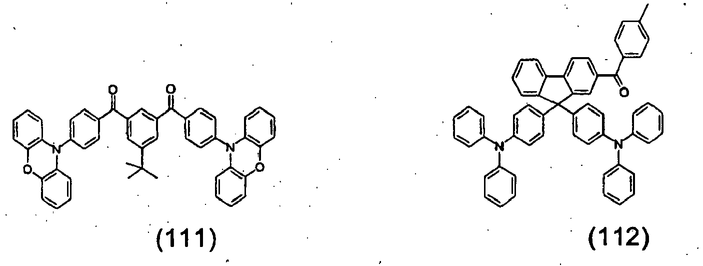

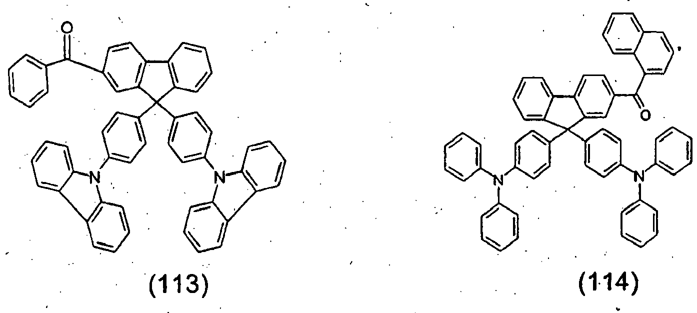

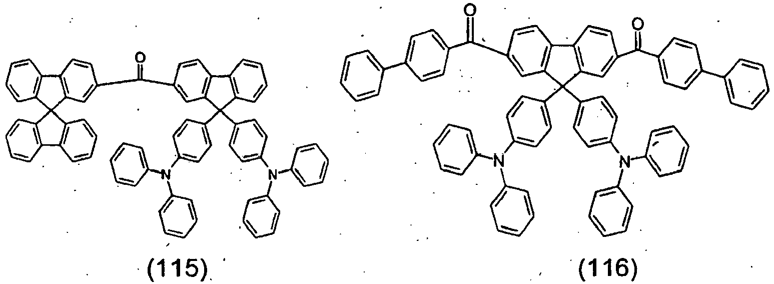

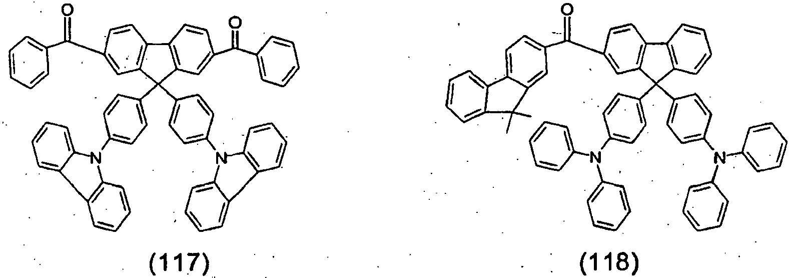

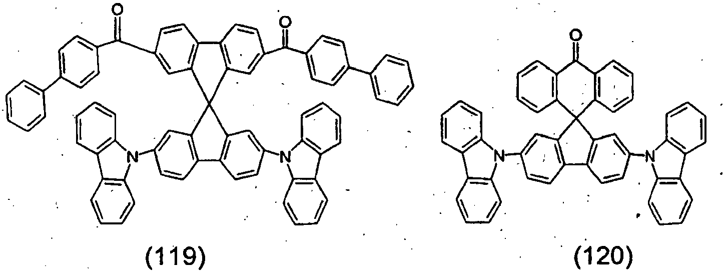

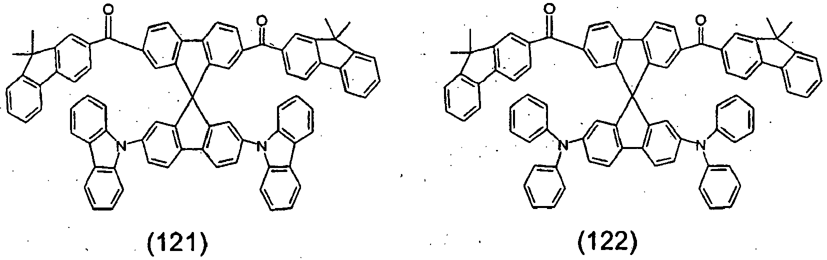

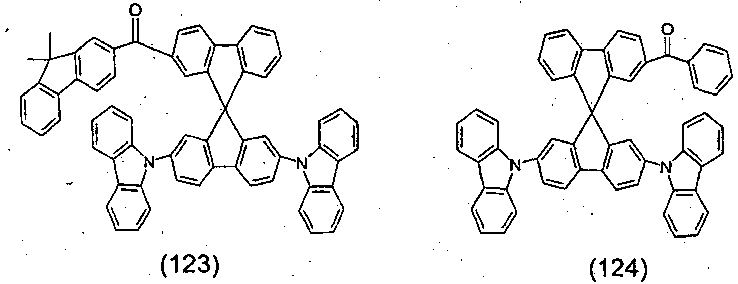

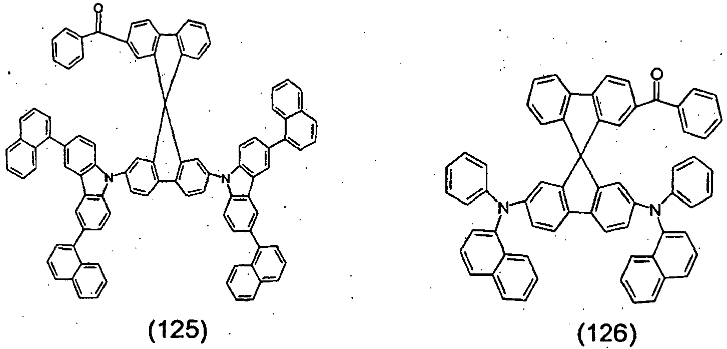

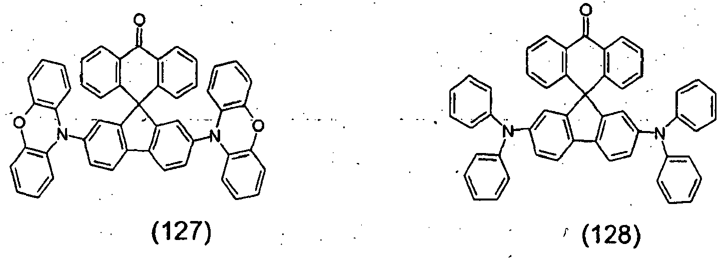

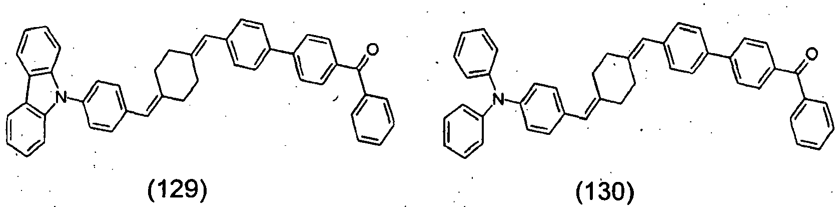

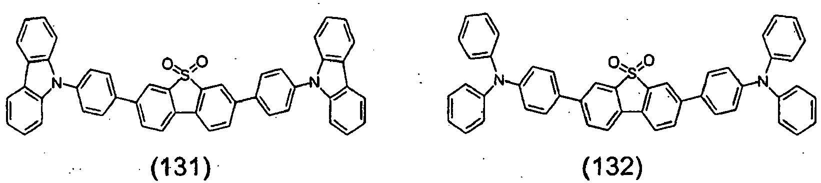

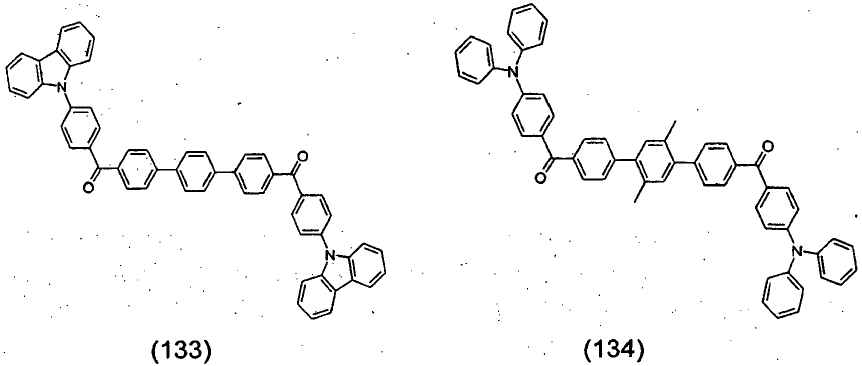

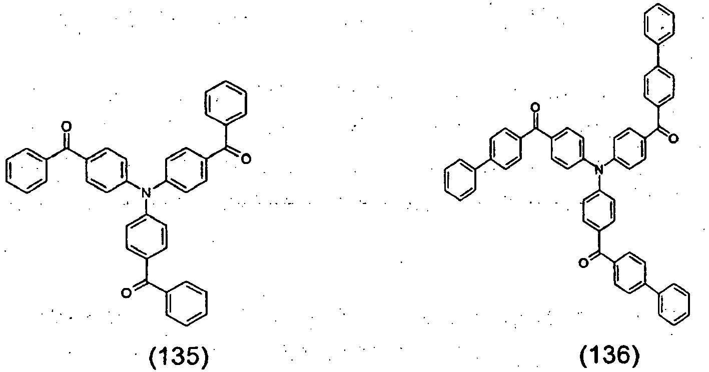

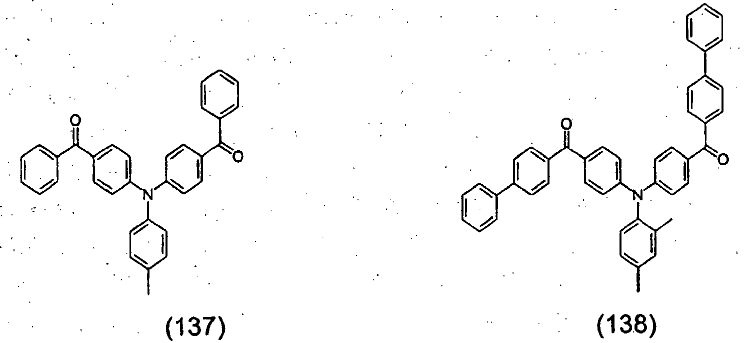

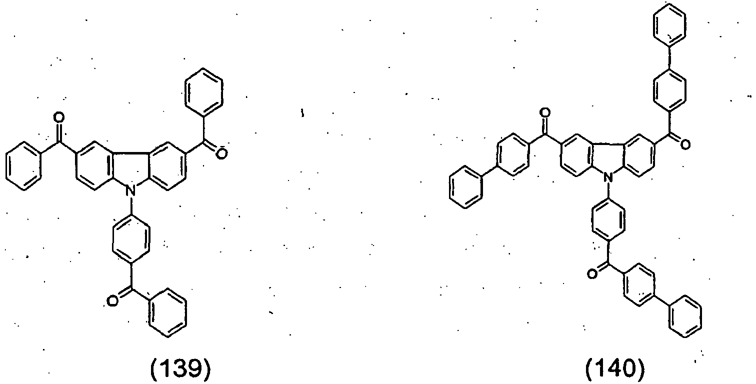

Die erfindungsgemäßen Verbindungen, insbesondere Verbindungen, welche mit reaktiven Abgangsgruppen, wie Brom, lod, Triflat, Tosylat, Boronsäure oder Boronsäureester substituiert sind, können auch als Monomere zur Erzeugung entsprechender Polymere, Oligomere oder auch als Kern von Dendrimeren Verwendung finden. Die Polymerisation erfolgt dabei bevorzugt über die Halogen- oder die Boronsäurefunktionalität.

Weiterer Gegenstand der Erfindung sind somit Polymere, Oligomere oder Dendrimere enthaltend eine oder mehrere Strukturelemente gemäß Formel (1), wobei ein oder mehrere Reste R¹ Bindungen des Strukturelements gemäß Formel (1) zum Polymer, Oligomer oder Dendrimer darstellen.

Für die Wiederholeinheiten gemäß Formel (1) bzw. gemäß den Formeln (4) bis (10) gelten dieselben Bevorzugungen wie oben beschrieben.

Diese Verbindungen werden homopolymerisiert oder mit weiteren Monomeren copolymerisiert. Geeignete und bevorzugte Comonomere sind gewählt aus Fluorenon (z. B. gemäß EP 842208 oder WO 00/22026), Spirobifluorenen (z. B. gemäß EP 707020, EP 894107 oder WO 06/061181), Para-phenylenen (z. B. gemäß WO 92/18552), Carbazolen (z. B. gemäß WO 04/070772 oder WO 04/113468), Thiophenen (z: B. gemäß EP 1028136), Dihydrophenanthrenen (z. B. gemäß WO 05/014689), cis- und trans-Indenofluorenen (z. B. gemäß WO 04/041901 oder WO 04/113412), Ketonen (z. B. gemäß WO 05/040302), Phenanthrenen (z. B. gemäß WO 05/104264 oder der nicht offen gelegten Anmeldung DE 102005037734.3) oder auch mehreren dieser Einheiten. Diese Polymere können auch noch weitere Einheiten enthalten, beispielsweise emittierende Einheiten, wie z. B. phosphoreszierende Metallkomplexe (z. B. gemäß WO 06/003000), und/oder Ladungstransporteinheiten, insbesondere solche basierend auf Triarylaminen. Die emittierenden Einheiten, beispielsweise die phosphoreszierenden Metallkomplexe, können auch zu dem Polymer zugemischt werden.

Die erfindungsgemäßen Verbindungen können nach Standardmethoden der organischen Chemie synthetisiert werden. So ist der Aufbau aromatischer Ketone beispielsweise durch Friedel-Crafts-Acylierung möglich. Weiterhin können aromatische Ketone durch Umsetzung eines aromatischen Nitrils mit einer aromatischen Organometallverbindung, beispielsweise einer Aryllithiumverbindung oder einem aromatischen Grignard-Reagenz, gefolgt von Hydrolyse des intermediär gebildeten Imins, möglich. Diese Ketone können funktionalisiert, beispielsweise bromiert, werden und in einem weiteren Schritt mit einem aromatischen Amin, beispielsweise mit Carbazol oder einem Diarylamin, in einer Hartwig-Buchwald-Kupplung zu den erfindungsgemäßen Verbindungen umgesetzt werden.

Ein weiterer Gegenstand der vorliegenden Erfindung ist daher ein Verfahren zur Synthese der erfindungsgemäßen Verbindungen durch Hartwig-Buchwald-Kupplung eines aromatischen, Ketons, weiches mit einem oder mehreren Halogenen, vorzugsweise Brom oder lod, oder einer Gruppe OSO₂R², vorzugsweise Triflat oder Tosylat, substituiert ist mit einer Diarylaminoverbindung, wobei die beiden Arylgruppen auch durch eine Gruppe E verbrückt sein können.

Weiterhin können die bromierten Verbindungen entweder direkt oder nach Überführung in ein Boronsäurederivat als Monomere zur Erzeugung von Polymeren, Oligomeren oder Dendrimeren eingesetzt werden.

Die erfindungsgemäßen Verbindungen und die entsprechenden Polymere, Oligomere und Dendrimere eignen sich für den Einsatz in organischen Elektrolumineszenzvorrichtungen (OLEDs, PLEDs), insbesondere als Triplett-Matrixmaterialien.

Ein weiterer Gegenstand der Erfindung ist daher die Verwendung von Verbindungen enthaltend mindestens ein Strukturelement gemäß Formel (1) bzw. Verbindungen gemäß den Formeln (4) bis (10) und entsprechender Polymere, Oligomere und Dendrimere in organischen elektronischen Vorrichtungen, insbesondere in organischen Elektrolumineszenzvorrichtungen.

Nochmals ein weiterer Gegenstand der Erfindung sind organische elektronische Vorrichtungen, enthaltend mindestens eine Verbindung enthaltend mindestens ein Strukturelement gemäß Formel (1) bzw. Verbindungen gemäß den Formeln (4) bis (10) bzw. mindestens ein entsprechendes Polymer,Oligomer oder Dendrimer, insbesondere organische Elektrolumineszenzvorrichtungen, enthaltend Anode, Kathode und mindestens eine emittierende Schicht, dadurch gekennzeichnet, dass mindestens eine Schicht mindestens eine Verbindung enthält, welche mindestens ein Strukturelement gemäß Formel (1) enthält, bzw. mindestens eine Verbindung gemäß den Formeln (4) bis (10).

Außer Kathode, Anode und der emittierenden Schicht kann die organische Elektrolumineszenzvorrichtung noch weitere Schichten enthalten, beispielsweise jeweils eine oder mehrere Lochinjektionsschichten, Lochtransportschichten, Elektronentransportschichten, Elektroneninjektionsschichten und/oder Charge-Generation Layers (IDMC 2003, Taiwan; Session 21 OLED (5), T. Matsumoto, T. Nakada, J. Endo, K. Mori, N. Kawamura, A. Yokoi, J. Kido, Multiphoton Organic EL Device Having Charge Generation Layer). Ebenso können zwischen zwei emittierenden Schichten Interlayers eingebracht sein, welche beispielsweise eine Excitonen-blockierende Funktion aufweisen. Es sei aber darauf hingewiesen, dass nicht notwendigerweise jede dieser Schichten vorhanden sein muss.

In einer weiteren bevorzugten Ausführungsform der Erfindung enthält die organische Elektrolumineszenzvorrichtung mehrere emittierende Schichten, wobei mindestens eine Schicht mindestens eine erfindungsgemäße Verbindung bzw. mindestens ein entsprechendes Polymer, Oligomer oder Dendrimer enthält. Besonders bevorzugt weisen diese Emissionsschichten insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können und die gelbes, orange oder rotes Licht emittieren. Insbesondere bevorzugt sind Dreischichtsysteme, wobei mindestens eine dieser Schichten mindestens eine erfindungsgemäße Verbindung bzw. mindestens ein entsprechendes Polymer, Oligomer oder Dendrimer enthält und wobei die drei Schichten blaue, grüne und orange oder rote Emission zeigen (für den prinzipiellen Aufbau siehe z. B. WO 05/011013). Ebenso eignen sich für weiße Emission Emitter, welche breitbandige Emissionsbanden aufweisen und dadurch weiße Emission zeigen.

Um als Funktionsmaterial Verwendung zu finden, werden die erfindungsgemäßen Verbindungen oder deren Mischungen oder Polymere oder Dendrimere enthaltend Strukturelemente gemäß Formel (1) oder deren Mischungen, gegebenenfalls zusammen mit emittierenden Verbindungen und/oder auch weiteren Verbindungen, nach allgemein bekannten und dem Fachmann geläufigen Methoden, wie Vakuumverdampfung, Verdampfen im Trägergasstrom oder aus Lösung durch Spincoaten oder mit verschiedenen Druckverfahren (z. B. Tintenstrahldrucken, Off-set-Drucken, LITI-Druck, etc.), in Form eines Films auf ein Substrat aufgebracht. Dabei kann die Verwendung von Druckverfahren und anderen lösungsbasierten Prozessen Vorteile hinsichtlich der Skalierbarkeit der Fertigung, wie auch bezüglich der Einstellung von Mischungsverhältnissen in verwendeten Blend-Schichten haben. Durch die gute Löslichkeit und die guten Filmbildungseigenschaften eignen sich die erfindungsgemäßen Verbindungen besonders gut auch zur Herstellung organischer elektronischer Vorrichtungen als Lösung.

In einer bevorzugten Ausführungsform der Erfindung werden die erfindungsgemäßen Verbindungen bzw. die entsprechenden Polymere, Oligomere oder Dendrimere als Matrix für phosphoreszierende Dotanden eingesetzt. Diese phosphoreszierenden Emitter enthalten mindestens eine Verbindung, die bei geeigneter Anregung Licht, vorzugsweise im sichtbaren Bereich, emittiert und außerdem mindestens ein Atom der Ordungszahl größer 20, bevorzugt größer 38 und kleiner 84, besonders bevorzugt größer 56 und kleiner 80 enthält. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium enthalten.

Besonders bevorzugte organische Elektrolumineszenzvorrichtungen enthalten als phosphorezierenden Emitter mindestens eine Verbindung der Formeln (18) bis (21), 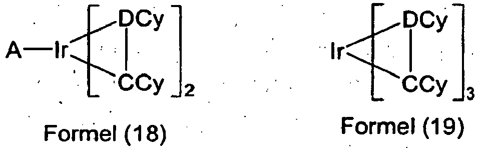 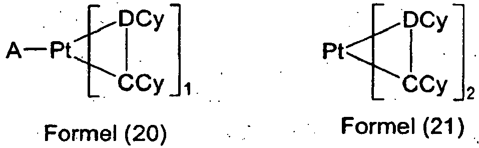 wobei R¹ dieselbe Bedeutung hat, wie oben beschrieben, und für die weiteren verwendeten Symbole gilt:
- DCy: ist gleich oder verschieden bei jedem Auftreten eine cyclische Gruppe, die mindestens ein Donoratom, bevorzugt Stickstoff oder Phosphor, enthält, über welches die cyclische Gruppe an das Metall gebunden ist, und die wiederum einen oder mehrere Substituenten R¹ tragen kann; die Gruppen DCy und CCy sind über eine kovalente Bindung miteinander verbunden;
- CCy: ist gleich oder verschieden bei jedem Auftreten eine cyclische Gruppe, die ein Kohlenstoffatom enthält, über welches die cyclische Gruppe an das Metall gebunden ist und die wiederum einen oder mehrere Substituenten R¹ tragen kann;
- A: ist gleich oder verschieden bei jedem Auftreten ein monoanionischer, zweizähnig chelatisierender Ligand, bevorzugt ein Diketonatligand.

Dabei kann durch Bildung von Ringsystemen zwischen mehreren Resten R¹ auch eine Brücke zwischen den Gruppen DCy und CCy vorliegen.

Beispiele der oben beschriebenen Emitter können den Anmeldungen WO 00/70655, WO 01/41512, WO 02/02714, WO 02/15645, EP 1191613, EP 1191612, EP 1191614 und WO 05/033244 entnommen werden. Generell eignen sich hier phosphoreszierende Komplexe, wie sie gemäß dem Stand der Technik für phosphoreszierende OLEDs verwendet werden. Wenn die OLED aus Lösung hergestellt werden soll, sollten die Emitter entsprechend substituiert sein, dass auch sie eine ausreichende Löslichkeit für die Verarbeitung aus Lösung aufweisen.

Die erfindungsgemäße Mischung enthält zwischen 1 und 99 Gew.-%, vorzugsweise zwischen 3 und 95 Gew.-%, besonders bevorzugt zwischen 5 und 50 Gew.-%, insbesondere zwischen 7 und 20 Gew.-% des phosphoreszierenden Emitters bezogen auf die Gesamtmischung aus Emitter und Matrixmaterial. Entsprechend enthält die erfindungsgemäße Mischung zwischen 99 und 1 Gew.-%, vorzugsweise zwischen 97 und 5 Gew.-%, besonders bevorzugt zwischen 95 und 50 Gew.-%, insbesondere zwischen 93 und 80 Gew.-% des erfindungsgemäßen Matrixmaterials bezogen auf die Gesamtmischung aus Emitter und Matrixmaterial.

In einer weiteren bevorzugten Ausführungsform der Erfindung wird die erfindungsgemäße Verbindung in einer Zwischenschicht (Interlayer) zwischen einer fluoreszierenden und einer phosphoreszierenden emittierenden Schicht eingebracht, insbesondere zwischen einer blau fluoreszierenden und einer grün, gelb, orange oder rot phosphoreszierenden Schicht. Die Verwendung von erfindungsgemäßen Verbindungen in einer solchen Interlayer führt zu Effizienzsteigerung der OLED. Wenn die Verbindung gemäß Formel (1) bzw. gemäß Formel (4) bis (10) in einer solchen Interlayer verwendet wird, wird sie bevorzugt als Reinsubstanz verwendet.

Weiterhin bevorzugt ist eine organische Elektrolumineszenzvorrichtung, dadurch gekennzeichnet; dass eine oder mehrere Schichten mit einem Sublimationsverfahren beschichtet werden. Dabei werden die Materialien in Vakuum-Sublimationsanlagen bei einem Druck kleiner 10⁻⁵ mbar, bevorzugt kleiner 10⁻⁶ mbar, besonders bevorzugt kleiner 10⁻⁷ mbar aufgedampft.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht.

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck oder Offsetdruck, besonders bevorzugt aber LITI (Light Induced Thermal Imaging, Thermotransferdruck) oder Ink-Jet Druck (Tintenstrahldruck), hergestellt werden. Da die erfindungsgemäßen Verbindungen eine hohe Löslichkeit in gängigen organischen Lösemitteln und gute Filmbildungseigenschaften aufweisen, eignen sie sich auch besonders gut zur Verarbeitung aus Lösung.

Die erfindungsgemäßen Verbindungen weisen bei Verwendung in organischen Elektrolumineszenzvorrichtungen folgende überraschende Vorteile gegenüber dem Stand der Technik auf:
1. Die Ladungsbalance der mit den erfindungsgemäßen Verbindungen hergestellten OLEDs ist besser ausgeglichen als mit OLEDs gemäß dem Stand der Technik. Dies führt zu verringerten Betriebsspannungen und damit höheren Effizienzen.
2. Auch die Lebensdauer der Vorrichtungen verbessert sich.
3. Die erfindungsgemäßen Verbindungen sind gut in den gängigen organischen Lösemitteln löslich. Weiterhin weisen sie bei Verarbeitung aus Lösung sehr gute Filmbildungseigenschaften auf. Dadurch wird insbesondere eine lösungsbasierte Verarbeitung von Mischungen der erfindungsgemäßen Verbindungen mit löslichen Triplett-Emittern ermöglicht.
4. Die Verwendung der erfindungsgemäßen Verbindungen in einer Zwischenschicht (Interlayer) zwischen einer fluoreszierenden und einer phosphoreszierenden emittierenden Schicht führt zu einer Effizienzsteigerung der organischen Elektrolumineszenzvorrichtung.

Im vorliegenden Anmeldetext wird auf die Verwendung der erfindungsgemäßen Verbindungen in Bezug auf OLEDs und PLEDs und die entsprechenden Displays abgezielt. Trotz dieser Beschränkung der Beschreibung ist es für den Fachmann ohne weiteres erfinderisches Zutun möglich, die erfindungsgemäßen Verbindungen auch für weitere Verwendungen in anderen elektronischen Vorrichtungen einzusetzen, z. B. für organische Feld-Effekt-Transistoren (O-FETs), organische Dünnfilmtransistoren (O-TFTs), organische lichtemittierende Transistoren (O-LETs), organische integrierte Schaltungen (O-ICs), organische Solarzellen (O-SCs), organische Feld-Quench-Devices (O-FQDs), lichtemittierende elektrochemische Zellen (LECs), organische Laserdioden (O-Laser) oder organischen Photorezeptoren.

Die Verwendung der erfindungsgemäßen Verbindungen in den entsprechenden Vorrichtungen ebenso wie diese Vorrichtungen selbst sind ebenfalls ein Gegenstand der vorliegenden Erfindung.

Die Erfindung wird durch die nachfolgenden Beispiele näher erläutert, ohne sie dadurch einschränken zu wollen.

### Beispiele:

Die nachfolgenden Synthesen werden, sofern nicht anders angegeben, unter einer Schutzgasatmosphäre in getrockneten Lösungsmitteln durchgeführt. Die Edukte werden von ALDRICH [Kupfer(I)cyanid, Acetylchlorid, N-Methylpyrrolidinon (NMP)] bezogen. 2-Brom-9,9'-spiro-bifluoren (J. Pei et al., J. Org. Chem. 2002, 67(14), 4924-4936) wird nach Literaturmethode dargestellt. 2-Cyano-9,9'-spirobifluoren wird gemäß WO 04/093207 synthetisiert.

### Beispiel 1: Synthese von Verbindung M1

### a) 2,7-Dibrom-2'-cyano-9,9'-spirobifluoren

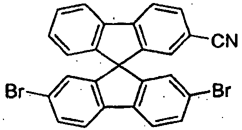

80.0 g (234 mmol) 2-Cyano-9,9'-spirobifluoren werden in 800 ml Dichlor-methan gelöst. 69.0 g (5.62 mol) Na₂CO₃ und 660 ml Wasser werden zugegeben. Es werden 80 ml (0.97 mol) Brom bei 50 °C zugetropft und 16 h bei Raumtemperatur gerührt. Die Reaktionsmischung wird bei 0 °C unter Rühren mit gesättigter Natriumsulfitlösung bis zur Entfärbung versetzt. Die organische Phase wird mit Dichlormethan verdünnt, mit gesättigter Natriumsulfitlösung und Wasser gewaschen, über Magnesiumsulfat getrocknet und bis zur Trockene eingeengt. Das Rohprodukt wird mehrmals aus Toluol umkristallisiert. Nach Trocknen der Kristalle im Vakuum bei 50 °C werden 76.5 g 97%iger Reinheit, entsprechend 65.4 % d. Th., erhalten.

### b) (2,7-Dibrom-9,9'-spirobifluoren-2'-yl)(9,9'-spirobifluoren-2-yl)keton

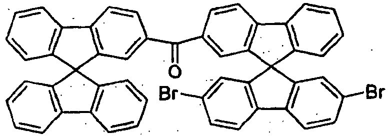

Aus einer Lösung von 40.0 g (101 mmol) 2-Brom-9,9'-spirobifluoren und 1 ml 1,2-Dichlorethan in 30 ml 1,2-Dimethoxyethan und 300 ml THF und 2.8 g (115 mmol) Magnesium wird in der Siedehitze das entsprechende Grignard-Reagenz hergestellt. Zu dieser Grignard-Lösung wird bei 0-5 °C eine Lösung von 50.6 g (101 mmol) 2,7-Dibrom-2'-cyano-9,9'-spirobifluoren in einer Mischung aus 130 ml THF und 130 ml Toluol während 15 min. zugetropft. Anschließend wird die Mischung 16 h unter Rückfluss erhitzt. Nach Abkühlen wird die Reaktionsmischung bis zur Trockene eingeengt. Der Feststoff wird in 1100 ml NMP aufgenommen und mit 40 ml Wasser und 0.05 ml Eisessig 24 h unter Rückfluss erhitzt. Es werden eine Mischung aus 600 ml Methanol und 600 ml 1N Salzsäure zugesetzt und der ausfallende Feststoff durch Filtration abgetrennt und getrocknet. Das Rohprodukt wird mehrmals aus Dioxan umkristallisiert. Die Ausbeute bei einer Reinheit > 97 % nach HPLC beträgt 41.5 g, entsprechend 50.2 % d. Th..

### c) Synthese von Verbindung M1

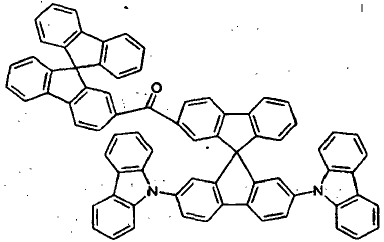

39.0 g (223 mmol) Carbazol, 75.5 g (92.5 mmol) (2,7-Dibrom-9,9'-spiro-bifluoren-2'-yl)(9,9'-spirobifluoren-2-yl)keton und 128.0 g (0.555 mol) Rubidiumcarbonat werden in 2500 ml Xylol suspendiert. Anschließend wird Tri-tert-butylphosphin und Palladiumacetat zugesetzt. Die Reaktionsmischung wird 40 h unter Rückfluss gerührt Die Reaktionsmischung wird nach dem Abkühlen mit Wasser und 1 N Salzsäure mehrmals gewaschen. Die vereinigten organischen Phasen werden über Magnesiumsulfat getrocknet und das Lösemittel am Rotationsverdampfer entfernt. Das Rohprodukt wird mehrmals aus Toluol umkristallisiert. Die Ausbeute bei einer Reinheit > 99.99 % nach HPLC beträgt 58.4 g, entsprechend 31.8 % d. Th. T_{G} = 213 °C, Tₘ = 350 °C.

### Beispiel 2: Synthese von Verbindung M2

### a) Synthese von Tris-p-bromphenylphosphinoxid

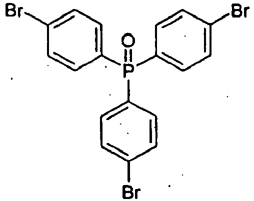

Die Herstellung dieser Verbindung ist in Journal of Fluorine Chemistry 2003, 124, 45-54 beschrieben.

### b) Synthese von Tris-p-(9H-Carbazole)phenylphosphinoxid (M2)

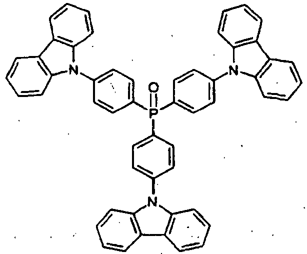

Eine gut gerührte, entgaste Suspension aus 33.8 g (315 mmol) Tris-p-bromphenyl-phosphinoxid, 52.6 g (315 mmol) Carbazol und 140 g (1466 mmol) K₃PO₄ in Xylol wird mit 4.6 ml (18.7 mmol) Tri-*tert*-butylphosphin und 5 min. später mit 0.84 g (3.7 mmol) Palladium(II)acetat versetzt und 96 h unter Rückfluss erhitzt. Nach Erkalten wird die organische Phase abgetrennt, dreimal mit 200 ml Wasser und einmal mit 300 ml gesättigter, wässriger Kochsalzlösung gewaschen und anschließend über Magnesiumsulfat getrocknet. Nach Abfiltrieren des Trockenmittels wird die organische Phase im Vakuum zur Trockene einrotiert. Der so erhaltene gelbe Rückstand wird aus DMF umkristallisiert. Die ausgefallenen Kristalle werden abgesaugt, mit etwas MeOH gewaschen und anschließend im Vakuum getrocknet; Ausbeute: 24.6 g, 48.4 % d. Th.; Reinheit: 99.9% n. HPLC. T_{G} = 170 °C, Tₘ = 322 °C.

### Beispiel 3: Synthese von M3

### a) Synthese von 1,3-Bis(4-brombenzoyl)-5-tert-butylbenzol

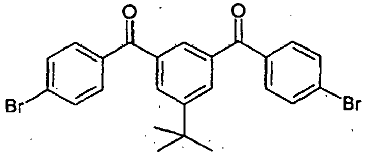

Die Herstellung dieser Verbindung ist in Macromolecules 2004, 32, 8269-8277 beschrieben.

### b) Synthese von 1,3-Bis[4-(diphenylamino)benzoyl-5-tert-butyl]benzol (M3)

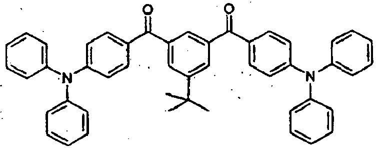

Eine gut gerührte, entgaste Suspension aus 67 g (133 mmol) 1,3-Bis(4-brombenzoyl-5-*tert*-butyl)benzol, 56.2 g (332.5 mmol) Diphenylamin und 36 g (373.6 mmol) NaO^{t}Bu in Toluol wird mit 3.5 ml (14.9 mmol) Tri-*tert-*butylphosphin und 5 min. später mit 0.70 g (3.15 mmol) Palladium(II)-acetat versetzt und 8 h unter Rückfluss erhitzt. Nach Erkalten wird die organische Phase abgetrennt, dreimal mit 200 ml Wasser und einmal mit 300 ml gesättigter, wässriger Kochsalzlösung gewaschen und anschließend über Magnesiumsulfat getrocknet. Nach Abfiltrieren des Trockenmittels wird die organische Phase im Vakuum zur Trockene einrotiert. Der so erhaltene gelbe Rückstand wird aus DMF umkristallisiert. Die ausgefallenen Kristalle werden abgesaugt, mit etwas MeOH gewaschen und anschließend im Vakuum getrocknet; Ausbeute: 43.1 g, 48 % d. Th.; Reinheit: 99.9 % n. HPLC. T_{G} = 96.1 °C, Tₘ = 196.8 °C.

### Beispiel 4: Synthese von M4

### a) Synthese von 1,3,5-Tris(4-brombenzoyl)benzol

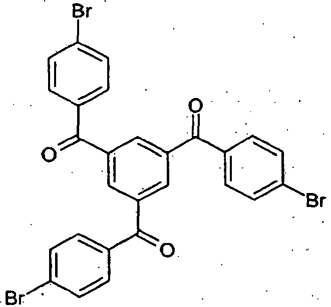

Die Herstellung dieser Verbindung ist in Synthesis 2003, 15, 2301-2303 beschrieben.

### b) 1,3,5-Tris[4-(diphenylamino)benzoyl]benzol (M4)

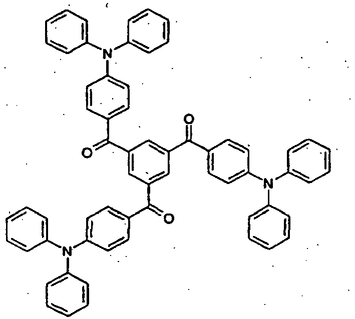

Eine gut gerührte, entgaste Suspension aus 67 g (106.8 mmol) 1,3,5-Tris(4-brombenzoyl)benzol, 68.7 g (405.9 mmol) Diphenylamin und 42.4 g (441.2 mmol) NaO^{t}Bu in Toluol wird mit 1.3 ml (5,38 mmol) Tri-*tert*-butylphosphin und 5 min. später mit 0.72 g (3.2 mmol) Palladium(II)acetat versetzt und 8 h unter Rückfluss erhitzt. Nach Erkalten wird die organische Phase abgetrennt, dreimal mit 200 ml Wasser und einmal mit 300 ml gesättigter, wässriger Kochsalzlösung gewaschen und anschließend über Magnesiumsulfat getrocknet. Nach Abfiltrieren des Trockenmittels wird die organische Phase im Vakuum zur Trockene einrotiert. Der so erhaltene gelbe Rückstand wird aus DMF umkristallisiert. Die ausgefallenen Kristalle werden abgesaugt, mit etwas MeOH gewaschen und anschließend im Vakuum getrocknet; Ausbeute: 35 g, 37.4 % d. Th.; Reinheit: 99.9 % n. HPLC. T_{G} = 114 °C, Tₘ = 207 °C.

### Beispiel 5: Herstellung und Charakterisierung von organischen Elektrolumineszenzvorrichtungen, die die erfindungsgemäßen Verbindungen enthalten

Erfindungsgemäße Elektrolumineszenzvorrichtungen können, wie beispielsweise in WO 05/003253 beschrieben, dargestellt werden. Hier werden die Ergebnisse verschiedener OLEDs gegenübergestellt. Der grundlegende Aufbau, die verwendeten Materialien, der Dotierungsgrad und ihre Schichtdicken sind zur besseren Vergleichbarkeit identisch. Es wird ausschließlich der Host in der Emissionsschicht variiert. Das erste Beispiel beschreibt einen Vergleichsstandard nach dem Stand der Technik, bei dem die Emissionsschicht aus dem Wirtsmaterial BAIq und dem Gastmaterial (Dotanden) Ir(piq)₃ besteht. Des Weiteren wird eine OLED mit einer Emitterschicht bestehend aus den Wirtsmaterialien M1 bis M4 und dem Gastmaterial (Dotand) Ir(piq)₃ und beschrieben. Analog dem o. g. allgemeinen Verfahren, werden OLEDs mit folgendem Aufbau erzeugt:

| | |
|---|---|
| Lochinjektionsschicht (HIL) | 20 nm 2,2',7,7'-Tetrakis(di-para-tolylamino)-spiro-9,9'-bifluoren. |
| Lochtransportschicht (HTL) | 20 nm NPB (N-Naphthyl-N-phenyl-4,4'-diaminobiphenyl). |
| Emissionschicht (EML) | Host: BAIq (aufgedampft; bezogen von SynTec, Bis(2-methyl-8-chinolinolato)(paraphenylphenolato)aluminium(III)) als Vergleich oder **M1** bis **M4.** |
| | Dotand: Ir(piq)₃ (10 % Dotierung, aufgedampft; synthetisiert nach WO 03/0068526). |
| Lochblockierschicht (HBL) | BAIq 10 nm (bezogen von SynTec, Bis(2-methyl-8-chinolinolato)(para-phenyl-phenolato)aluminium(III)). |
| Elektronenleiter (ETL) | 20 nm AlQ₃ (bezogen von SynTec, Tris(chinolinato)aluminium(III)). |
| Kathode | 1 nm LiF, darauf 150 nm Al. |

Die Struktur von Ir(piq)₃ ist der Übersichtlichkeit halber im Folgenden abgebildet:

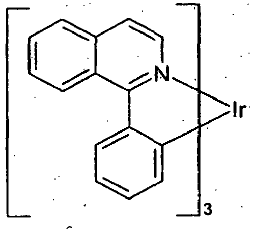

Diese noch nicht optimierten OLEDs werden standardmäßig charakterisiert; hierfür werden die Elektrolumineszenzspektren, die Effizienz (gemessen in cd/A) in Abhängigkeit von der Helligkeit, berechnet aus Strom-Spannungs-Helligkeit-Kennlinien (IUL-Kennlinien), und die Lebensdauer bestimmt.

Mit OLEDs hergestellt mit dem Standard-Host BAIq erhält man unter den oben beschriebenen Bedingungen typischerweise eine maximale Effizienz von etwa 8.1 cd/A bei Farbkoordinaten von CIE: x = 0.68, y = 0.32. Für die Referenzleuchtdichte von 1000 cd/m² werden Spannungen von 7.2 V benötigt. Die Lebensdauer beträgt etwa 6300 h bei einer Anfangsleuchtdichte von 1000 cd/m² (s. Tabelle 1). Im Gegensatz dazu zeigen OLEDs hergestellt mit den erfindungsgemäßen Hosts **M1** bis **M4** bei ansonsten gleichem Aufbau maximale Effizienzen von bis zu 8.9 cd/A bei Farbkoordinaten von CIE: x = 0.68, y = 0.32, wobei die benötigte Spannung für die Referenzleuchtdichte von 1000 cd/m² bei 5.6 V liegt (s. Tabelle 1). Die Lebensdauer bei einer Anfangsleuchtdichte von 1000 cd/m² ist mit 7900 h höher als mit dem Referenzmaterial BAIq (s. Tabelle 1).

**Tabelle 1: Device-Ergebnisse mit erfindungsgemäßen Hostmaterialien mit Ir(piq)₃ als Dotand**

| Experiment | EML | Max. Effizienz [cd/A] | Spannung [V] bei 1000 cd/m² | CIE (x, y) | Lebensdauer [h] Anfangshelligkeit 1000 [cd/m²] |
|---|---|---|---|---|---|
| Beispiel 6 (Vergleich) | BAIq:10% Ir(piq)₃ (30 nm) | 8.1 | 7.2 | 0.68/0.32 | 6300 |
| Beispiel 7 | **M1** :10% Ir(piq)₃ (30 nm) | 8.5 | 6.0 | 0.68/0.32 | 7500 |
| Beispiel 8 | **M2** : 10% Ir(piq)₃ (30 nm) | 8.5 | 5.8 | 0.68/0.32 | 7900 |
| Beispiel 9 | **M3** : 10% Ir(piq)₃ (30 nm) | 8.7 | 5.6 | 0.68/0.32 | 8200 |
| Beispiel 10 | **M4** : 10% Ir(piq)₃ (30 nm) | 8.9 | 5.7 | 0.68/0.32 | 7800 |

## Patentansprüche

1. Verbindungen enthaltend mindestens ein Strukturelement gemäß Formel (1), 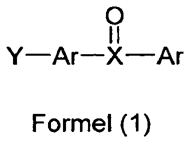 wobei für die verwendeten Symbole und Indizes gilt:
X ist bei jedem Auftreten gleich oder verschieden C, P(Ar) oder P(Ar-Y);
Ar ist bei jedem Auftreten gleich oder verschieden ein aromatisches oder heteroaromatisches Ringsystem mit 5 bis 60 aromatischen Ringatomen, welches mit einem oder mehreren Resten R¹ substituiert sein kann;
R¹ ist bei jedem Auftreten gleich oder verschieden H, F, Cl, Br, I, N(Ar¹)₂, CN, NO₂, Si(R²)₃, B(OR²)₂, C(=O)Ar¹, P(=O)(Ar¹)₂, S(=O)Ar¹, S(=O)₂Ar¹, CR²=CR²(Ar¹), Tosylat, Triflat, OSO₂R², eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten R² substituiert sein kann und wobei jeweils eine oder mehrere nicht benachbarte CH₂-Gruppen durch R²C=CR², C≡C, Si(R²)₂, Ge(R²)₂, Sn(R²)₂, C=O, C=S, C=Se, C=NR², P(=O)(R²), SO, SO₂, NR², O, S oder CONR² ersetzt sein können und wobei jeweils ein oder mehrere H-Atome durch F, Cl, Br, I, CN oder NO₂ ersetzt sein können, oder ein aromatisches oder heteroaromatisches Ringsystem mit 5 bis 60 aromatischen Ringatomen, das jeweils durch einen oder mehrere Reste R² substituiert sein kann, oder eine Aryloxy- oder Heteroaryloxygruppe mit 5 bis 60 aromatischen Ringatomen, die durch einen oder mehrere Reste R² substituiert sein kann, oder eine Kombination dieser Systeme; dabei können zwei oder mehrere Substituenten R¹ auch miteinander ein mono- oder polycyclisches aliphatisches oder aromatisches Ringsystem bilden;
Ar¹ ist bei jedem Auftreten gleich oder verschieden ein aromatisches oder heteroaromatisches Ringsystem mit 5 bis 60 aromatischen Ringatomen, welches mit einem oder mehreren Resten R² substituiert sein kann;
R² ist bei jedem Auftreten gleich oder verschieden H, F oder ein aliphatischer, aromatischer und/oder heteroaromatischer Kohlenwasserstoffrest mit 1 bis 20 C-Atomen, in dem auch H-Atome durch F ersetzt sein können; dabei können zwei oder mehrere Substituenten R² auch miteinander ein mono- oder polycyclisches aliphatisches oder aromatisches Ringsystem bilden;
Y ist bei jedem Auftreten gleich oder verschieden eine Gruppe der Formel (2) oder Formel (3), 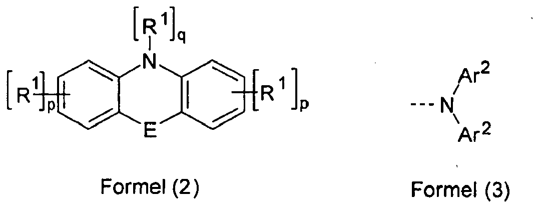 wobei die Einheit gemäß Formel (2) über eine beliebige Position mit Ar verknüpft ist und die Einheit gemäß Formel (3) über N mit Ar verknüpft ist, wobei R¹ die oben aufgeführte Bedeutung hat und weiterhin gilt:
E steht für O, S, N(R¹), P(R¹), P(=O)R¹, C(R¹)₂, Si(R¹)₂ oder für eine Einfachbindung;
Ar² ist gleich oder verschieden bei jedem Auftreten eine Aryl- oder Heteroarylgruppe mit 5 bis 20 aromatischen Ringatomen oder eine Triarylamingruppe mit 15 bis 30 aromatischen Ringatomen, welche jeweils mit einem oder mehreren Resten R¹ substituiert sein kann, mit der Maßgabe, dass an mindestens einer Gruppe Ar² mindestens ein Substituent R¹ vorhanden ist, welcher für eine Alkyl- oder für eine Silylgruppe steht;
p ist bei jedem Auftreten gleich oder verschieden 0, 1, 2, 3 oder 4;
q ist 0 oder 1, wobei q = 0 ist, wenn die Einheit gemäß Formel (2) über den Stickstoff an Ar gebunden ist, und q = 1 ist, wenn die Einheit gemäß Formel (2) über ein anderes Atom als den Stickstoff an Ar gebunden ist;
mit der Maßgabe, dass die Gruppe Ar, welche an X und an Y bindet, nicht durchgängig konjugiert ist, wenn die Verbindung gemäß Formel (1) genau eine Carbonylfunktion aufweist;
ausgenommen ist die folgende Verbindung:

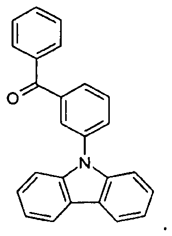

2. Verbindungen gemäß den Formeln (4), (5), (6), (7), (8), (9) und (10), 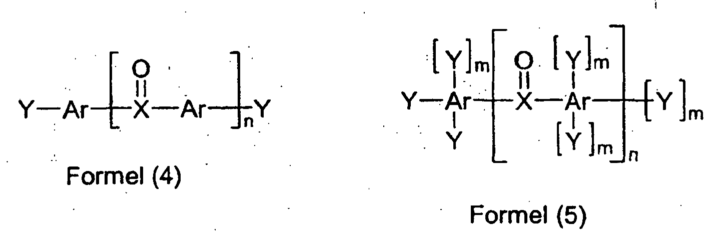 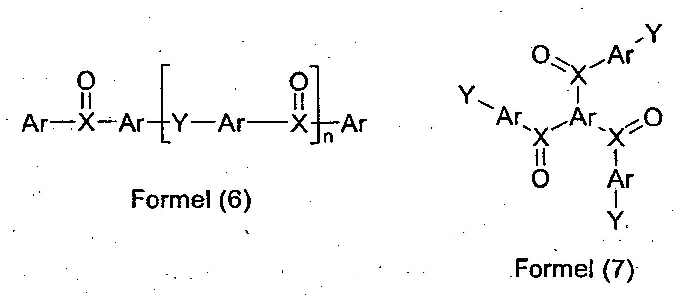 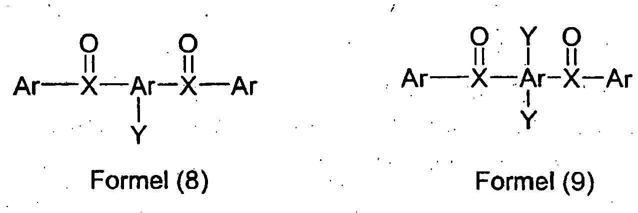 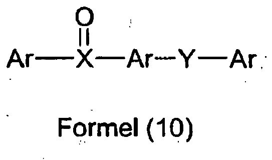 wobei die Symbole und Indizes die in Anspruch 1 aufgeführten Bedeutungen haben, und weiterhin gilt:
m ist bei jedem Auftreten gleich oder verschieden 0 oder 1, wobei m = 0 bedeutet, dass an Stelle von Y eine Gruppe R¹ gebunden ist;
n ist bei jedem Auftreten gleich oder verschieden 1, 2, 3, 4 oder 5.

3. Verbindungen nach Anspruch 1 oder 2, **dadurch gekennzeichnet, dass** die Verbindung mindestens zwei Gruppen X=O und/oder mindestens zwei Gruppen Y enthält.

4. Verbindungen nach einem oder mehreren der Ansprüche 1 bis 3, **dadurch gekennzeichnet, dass** das Verhältnis von Gruppen X=O zu Gruppen Y zwischen 1 : 10 und 10 : 1 beträgt.

5. Verbindungen nach einem oder mehreren der Ansprüche 1 bis 4, **dadurch gekennzeichnet, dass** die Gruppe Ar, welche gleichzeitig an X und an Y bindet, nicht durchgängig konjugiert ist.

6. Verbindungen nach einem oder mehreren der Ansprüche 1 bis 5, **dadurch gekennzeichnet, dass** die Gruppen Ar nur Phenyl- und/oder Naphthylgruppen, jedoch keine größeren kondensierten aromatischen Systeme, enthalten.

7. Verbindungen nach einem oder mehreren der Ansprüche 1 bis 6, **dadurch gekennzeichnet, dass** die Gruppen Ar, welche an X und an Y gebunden sind, gewählt sind aus den Einheiten gemäß Formel (11) bis Formel (17), 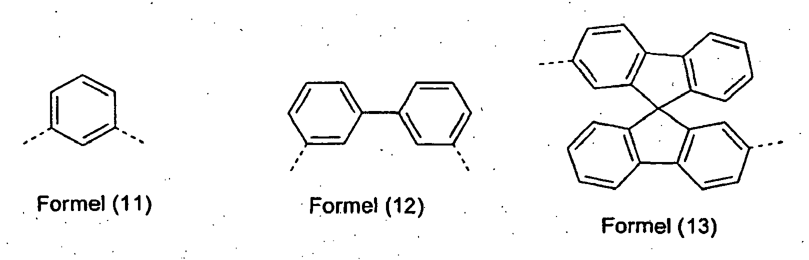 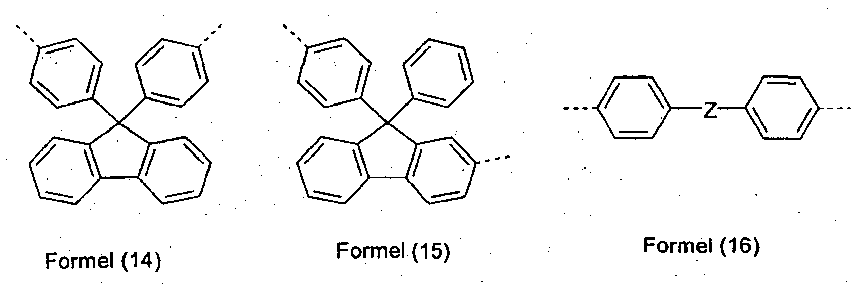 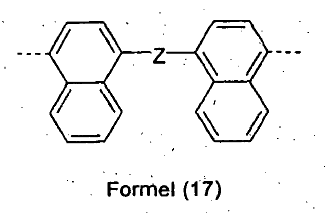 wobei die gestrichelte Bindung jeweils die Verknüpfung mit X und mit Y andeutet, wobei die Einheiten jeweils durch einen oder mehrere Reste R¹ substituiert sein können und weiterhin gilt:
Z ist bei jedem Auftreten gleich oder verschieden -[C(R¹)₂]ₖ-, Si(R¹)₂, O oder S;
k ist 1, 2, 3, 4, 5 oder 6.

8. Verbindungen nach einem oder mehreren der Ansprüche 1 bis 7, **dadurch gekennzeichnet, dass** die Einheiten Y bei jedem Auftreten gleich oder verschieden eine Gruppe der Formel (2a) bzw. (3a) sind, 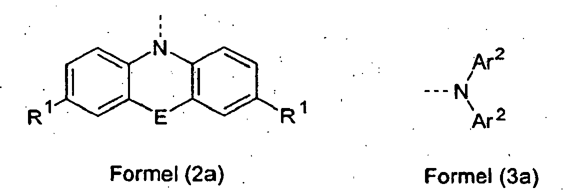 wobei die Einheiten über den Stickstoff mit Ar verknüpft sind, wobei R¹ die oben aufgeführte Bedeutung hat und weiterhin gilt:
E steht für eine Einfachbindung, O, S oder N(R¹);
Ar² ist gleich oder verschieden bei jedem Auftreten eine Aryl- oder Heteroarylgruppe mit 5 bis 10 aromatischen Ringatomen oder eine Triarylamingruppe mit 18 bis 24 aromatischen Ringatomen, welche jeweils mit einem oder mehreren Resten R¹ substituiert sein kann, mit der Maßgabe, dass an mindestens einer Gruppe Ar² mindestens ein Substituent R¹ vorhanden ist, welcher für eine Alkyl- oder für eine Silylgruppe steht.

9. Verbindungen nach einem oder mehreren der Ansprüche 1 bis 8, **dadurch gekennzeichnet, dass** das Symbol R¹ gleich oder verschieden bei jedem Auftreten für H, F, Br, N(Ar¹)₂, P(=O)(Ar¹)₂, C(=O)Ar¹, CR²=CR²Ar¹, eine geradkettige Alkylgruppe mit 1 bis 5 C-Atomen oder verzweigte Alkylgruppe mit 3 bis 5 C-Atomen, wobei eine oder mehrere nicht benachbarte CH₂-Gruppen durch -R²C=CR²- oder -O-ersetzt sein können und wobei ein oder mehrere H-Atome durch F ersetzt sein können, oder eine Arylgruppe mit 6 bis 16 C-Atomen oder Heteroarylgruppe mit 2 bis 16 C-Atomen oder eine Spirobifluorenyl-gruppe, die jeweils mit einem oder mehreren Resten R² substituiert sein kann, oder eine Kombination aus zwei oder drei dieser Systeme steht, oder dass das Symbol R¹ auch für eine geradkettige oder verzweigte Alkylgruppe mit bis zu 10 C-Atomen steht, falls die Struktureinheit gemäß Formel (1) in Polymeren verwendet oder aus Lösung verarbeitet wird.

10. Polymere, Oligomere oder Dendrimere enthaltend eine oder mehrere Einheiten nach einem oder mehreren der Ansprüche 1 bis 9, wobei ein oder mehrere Reste R¹ Bindungen der Verbindung gemäß Formel (1) zum Polymer, Oligomer oder Dendrimer darstellen.

11. Verfahren zur Herstellung von Verbindungen nach einem oder mehreren der Ansprüche 1 bis 9, **dadurch gekennzeichnet, dass** die Verbindung durch Hartwig-Buchwald-Kupplung eines aromatischen Ketons, welches mit einem oder mehreren Halogen bzw. einer oder mehreren Gruppen OSO₂R² substituiert ist, mit einer Diarylaminogruppe, wobei die beiden Arylgruppen auch durch eine Gruppe E verbrückt sein können, synthetisiert wird.

12. Verwendung von Verbindungen nach einem oder mehreren der Ansprüche 1 bis 10 in organischen elektronischen Vorrichtungen.

13. Vorrichtung, enthaltend Anode, Kathode und mindestens eine organische Schicht, **dadurch gekennzeichnet, dass** mindestens eine Schicht mindestens eine Verbindung nach einem oder mehreren der Ansprüche 1 bis 10 enthält, bevorzugt eine organische Elektrolumineszenzvorrichtung.

14. Organische Elektrolumineszenzvorrichtung nach Anspruch 13, **dadurch gekennzeichnet, dass** die Verbindung nach einem oder mehreren der Ansprüche 1 bis 10 in einer emittierenden Schicht als Matrix für phosphoreszierende Dotanden eingesetzt.

15. Organische Elektrolumineszenzvorrichtung nach Anspruch 14, **dadurch gekennzeichnet, dass** die phosphorezierenden Emitter ausgewählt sind aus Verbindungen der Formeln (18) bis (21), 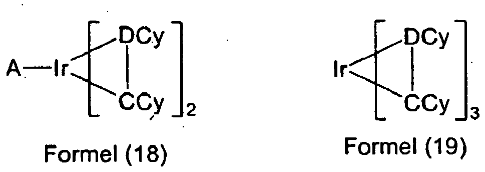 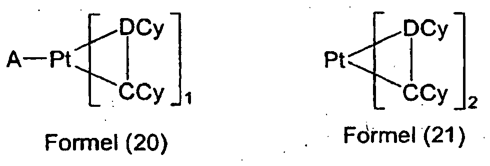 wobei R¹ dieselbe Bedeutung hat, wie oben beschrieben, und für die weiteren verwendeten Symbole gilt:
DCy ist gleich oder verschieden bei jedem Auftreten eine cyclische Gruppe, die mindestens ein Donoratom, bevorzugt Stickstoff oder Phosphor, enthält, über welches die cyclische Gruppe an das Metall gebunden ist, und die wiederum einen oder mehrere Substituenten R¹ tragen kann; die Gruppen DCy und CCy sind über eine kovalente Bindung miteinander verbunden;
CCy ist gleich oder verschieden bei jedem Auftreten eine cyclische Gruppe, die ein Kohlenstoffatom enthält, über welches die cyclische Gruppe an das Metall gebunden ist und die wiederum einen oder mehrere Substituenten R¹ tragen kann;
A ist gleich oder verschieden bei jedem Auftreten ein monoanionischer, zweizähnig chelatisierender Ligand, bevorzugt ein Diketonatligand.

16. Organische Elektrolumineszenzvorrichtung nach einem oder mehreren der Ansprüche 13 bis 15, **dadurch gekennzeichnet, dass** die Verbindung nach einem oder mehreren der Ansprüche 1 bis 10 in einer Zwischenschicht zwischen einer fluoreszierenden und einer phosphoreszierenden emittierenden Schicht eingebracht wird.

## Claims

1. Compounds containing at least one structural element of the formula (1), 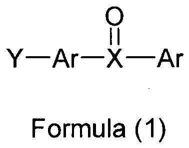 where the following applies to the symbols and indices used:
X is on each occurrence, identically or differently, C, P(Ar) or P(Ar-Y);
Ar is on each occurrence, identically or differently, an aromatic or heteroaromatic ring system having 5 to 60 aromatic ring atoms, which may be substituted by one or more radicals R¹;
R¹ is on each occurrence, identically or differently, H, F, Cl, Br, I, N(Ar¹)₂, CN, NO₂, Si(R²)₃, B(OR²)₂, C(=O)Ar¹, P(=O)(Ar¹)₂, S(=O)Ar¹, S(=O)₂Ar¹, CR²=CR²(Ar¹), tosylate, triflate, OSO₂R², a straight-chain alkyl, alkoxy or thioalkoxy group having 1 to 40 C atoms or a branched or cyclic alkyl, alkoxy or thioalkoxy group having 3 to 40 C atoms, each of which may be substituted by one or more radicals R² and where in each case one or more non-adjacent CH₂ groups may be replaced by R²C=CR², C=C, Si(R²)₂, Ge(R²)₂, Sn(R²)₂, C=O, C=S, C=Se, C=NR², P(=O)(R²), SO, SO₂, NR², O, S or CONR² and where in each case one or more H atoms may be replaced by F, Cl, Br, I, CN or NO₂, or an aromatic or heteroaromatic ring system having 5 to 60 aromatic ring atoms, which may in each case be substituted by one or more radicals R², or an aryloxy or heteroaryloxy group having 5 to 60 aromatic ring atoms, which may be substituted by one or more radicals R², or a combination of these systems; two or more substituents R¹ here may also form a mono- or polycyclic aliphatic or aromatic ring system with one another;
Ar¹ is on each occurrence, identically or differently, an aromatic or heteroaromatic ring system having 5 to 60 aromatic ring atoms, which may be substituted by one or more radicals R²;
R² is on each occurrence, identically or differently, H, F or an aliphatic, aromatic and/or heteroaromatic hydrocarbon radical having 1 to 20 C atoms, in which, in addition, H atoms may be replaced by F; two or more substituents R² here may also form a mono- or polycyclic aliphatic or aromatic ring system with one another;
Y is on each occurrence, identically or differently, a group of the formula (2) or formula (3), 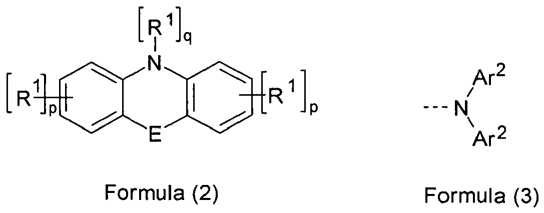 where the unit of the formula (2) is linked to Ar via any desired position and the unit of the formula (3) is linked to Ar via N, where R¹ has the meaning indicated above and furthermore:
E stands for O, S, N(R¹), P(R¹), P(=O)R¹, C(R¹)₂, Si(R¹)₂ or for a single bond;
Ar² is, identically or differently on each occurrence, an aryl or heteroaryl group having 5 to 20 aromatic ring atoms or a triarylamine group having 15 to 30 aromatic ring atoms, each of which may be substituted by one or more radicals R¹, with the proviso that at least one substituent R¹ which stands for an alkyl or silyl group is present on at least one group Ar²;
p is on each occurrence, identically or differently, 0, 1, 2, 3 or 4;
q is 0 or 1, where q = 0 if the unit of the formula (2) is bonded to Ar via the nitrogen and q = 1 if the unit of the formula (2) is bonded to Ar via an atom other than the nitrogen;
with the proviso that the group Ar which is bonded to X and to Y is not continuously conjugated if the compound of the formula (1) has precisely one carbonyl function;
with the exception of the following compound:

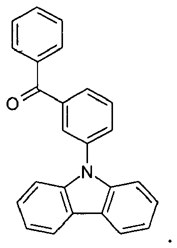

2. Compounds of the formulae (4), (5), (6), (7), (8), (9) and (10), 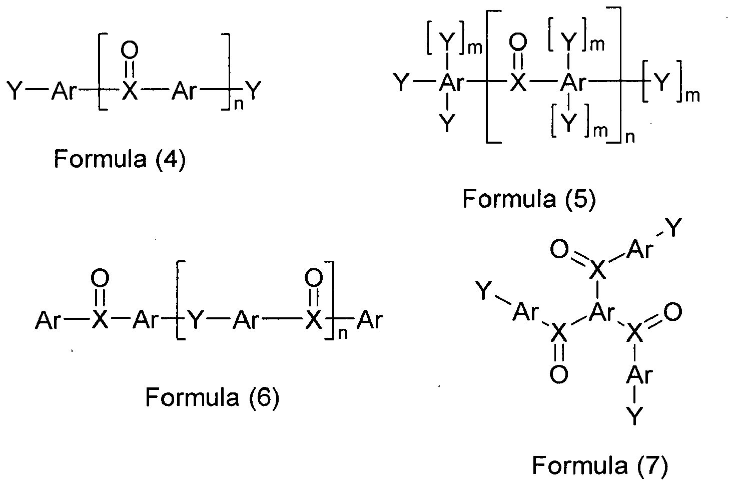 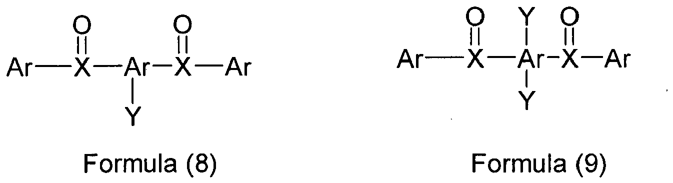 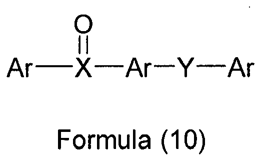 where the symbols and indices have the meanings indicated in Claim 1, and furthermore:
m is on each occurrence, identically or differently, 0 or 1, where m = 0 means that a group R¹ is bonded instead of Y;
n is on each occurrence, identically or differently, 1, 2, 3, 4 or 5.

3. Compounds according to Claim 1 or 2, **characterised in that** the compound contains at least two groups X=O and/or at least two groups Y.

4. Compounds according to one or more of Claims 1 to 3, **characterised in that** the ratio of groups X=O to groups Y is between 1 : 10 and 10 : 1.

5. Compounds according to one or more of Claims 1 to 4, **characterised in that** the group Ar which is simultaneously bonded to X and to Y is not continuously conjugated.

6. Compounds according to one or more of Claims 1 to 5, **characterised in that** the groups Ar only contain phenyl and/or naphthyl groups, but no larger condensed aromatic systems.

7. Compounds according to one or more of Claims 1 to 6, **characterised in that** the groups Ar which are bonded to X and to Y are selected from the units of the formula (11) to formula (17), 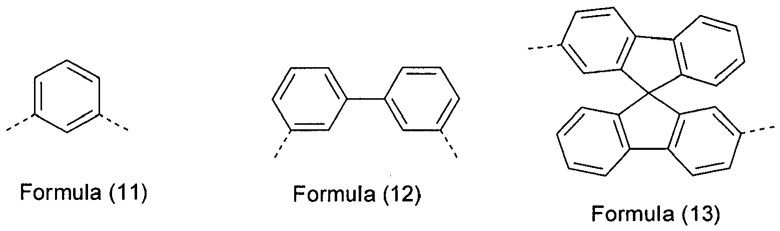 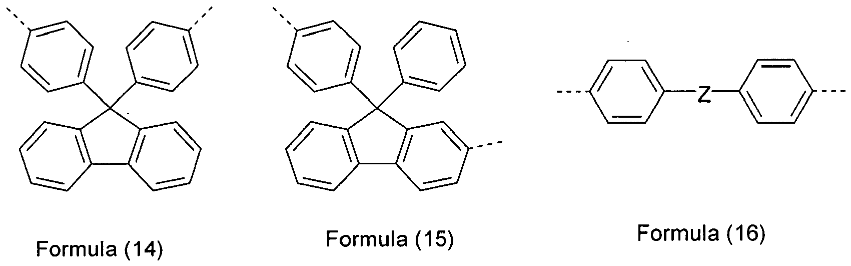 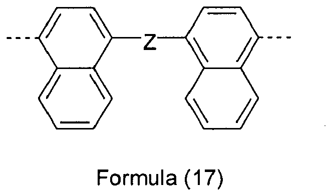 where the dashed bond in each case indicates the link to X and to Y, where the units may in each case be substituted by one or more radicals R¹ and furthermore:
Z is on each occurrence, identically or differently, -[C(R¹)₂]ₖ-, Si(R¹)₂, O or S;
k is 1, 2, 3, 4, 5 or 6.

8. Compounds according to one or more of Claims 1 to 7, **characterised in that** the units Y are on each occurrence, identically or differently, a group of the formula (2a) or (3a), 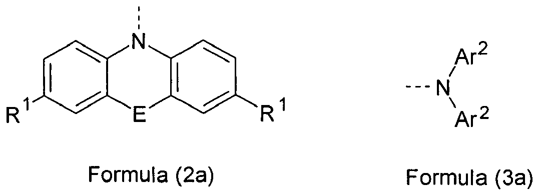 where the units are linked to Ar via the nitrogen, where R¹ has the meaning indicated above and furthermore:
E stands for a single bond, O, S or N(R¹);
Ar² is, identically or differently on each occurrence, an aryl or heteroaryl group having 5 to 10 aromatic ring atoms or a triarylamine group having 18 to 24 aromatic ring atoms, each of which may be substituted by one or more radicals R¹, with the proviso that at least one substituent R¹ which stands for an alkyl or silyl group is present on at least one group Ar².

9. Compounds according to one or more of Claims 1 to 8, **characterised in that** the symbol R¹ stands, identically or differently on each occurrence, for H, F, Br, N(Ar¹)₂, P(=O)(Ar¹)₂, C(=O)Ar¹, CR²=CR²Ar¹, a straight-chain alkyl group having 1 to 5 C atoms or branched alkyl group having 3 to 5 C atoms, where one or more non-adjacent CH₂ groups may be replaced by -R²C=CR²- or -O- and where one or more H atoms may be replaced by F, or an aryl group having 6 to 16 C atoms or heteroaryl group having 2 to 16 C atoms or a spirobifluorenyl group, each of which may be substituted by one or more radicals R², or a combination of two or three of these systems, or **in that** the symbol R¹ also stands for a straight-chain or branched alkyl group having up to 10 C atoms if the structural unit of the formula (1) is used in polymers or is processed from solution.

10. Polymers, oligomers or dendrimers containing one or more units according to one or more of Claims 1 to 9, where one or more radicals R¹ represent bonds from the compound of the formula (1) to the polymer, oligomer or dendrimer.

11. Process for the preparation of compounds according to one or more of Claims 1 to 9, **characterised in that** the compound is synthesised by Hartwig-Buchwald coupling of an aromatic ketone which is substituted by one or more halogens or one or more groups OSO₂R² to a diarylamino group, where the two aryl groups may also be bridged by a group E.

12. Use of compounds according to one or more of Claims 1 to 10 in organic electronic devices.

13. Device comprising anode, cathode and at least one organic layer, **characterised in that** at least one layer comprises at least one compound according to one or more of Claims 1 to 10, preferably an organic electroluminescent device.

14. Organic electroluminescent device according to Claim 13, **characterised in that** the compound according to one or more of Claims 1 to 10 is employed as matrix for phosphorescent dopants in an emitting layer.

15. Organic electroluminescent device according to Claim 14, **characterised in that** the phosphorescent emitters are selected from compounds of the formulae (18) to (21), 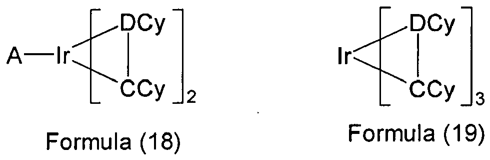 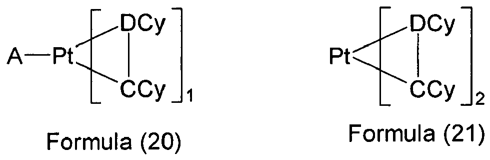 where R¹ has the same meaning as described above and the following applies to the other symbols used:
DCy is, identically or differently on each occurrence, a cyclic group which contains at least one donor atom, preferably nitrogen or phosphorus, via which the cyclic group is bonded to the metal, and which may in turn carry one or more substituents R¹; the groups DCy and CCy are connected to one another via a covalent bond;
CCy is, identically or differently on each occurrence, a cyclic group which contains a carbon atom via which the cyclic group is bonded to the metal and which may in turn carry one or more substituents R¹;
A is, identically or differently on each occurrence, a monoanionic, bidentate-chelating ligand, preferably a diketonate ligand.

16. Organic electroluminescent device according to one or more of Claims 13 to 15, **characterised in that** the compound according to one or more of Claims 1 to 10 is introduced in an interlayer between a fluorescent and a phosphorescent emitting layer.

## Revendications

1. Composés contenant au moins un élément structurel de la formule (1) : 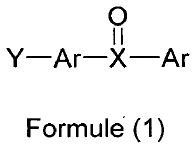 dans laquelle ce qui suit s'applique aux symboles et indices utilisés :
X est, pour chaque occurrence, de manière identique ou différente, C, P(Ar) ou P(Ar-Y) ;
Ar est, pour chaque occurrence, de manière identique ou différente, un système de cycle aromatique ou hétéroaromatique comportant 5 à 60 atomes de cycle aromatique, lequel peut être substitué par un radical ou plusieurs radicaux R^{1 ;}
R¹ est, pour chaque occurrence, de manière identique ou différente, H, F, Cl, Br, I, N(Ar¹)₂, CN, NO₂, Si(R²)₃, B(OR²)₂, C(=O)Ar¹, P(=O)(Ar¹)₂, S(=O)Ar¹, S(=O)₂Ar¹, CR²=CR²(Ar¹₎, tosylate, triflate, OSO₂R², un groupe alkyle, alcoxy ou thioalcoxy en chaîne droite comportant 1 à 40 atome(s) de C ou un groupe alkyle, alcoxy ou thioalcoxy ramifié ou cyclique comportant 3 à 40 atomes de C, dont chacun peut être substitué par un radical ou plusieurs radicaux R² et où, dans chaque cas, un ou plusieurs groupe(s) CH₂ non adjacents peut/peuvent être remplacé(s) par R²C=CR², C≡C, Si(R²)₂, Ge(R²)₂, Sn(R²)₂, C=O, C=S, C=Se, C=NR², P(=O)(R²), SO, SO₂, NR², O, S ou CONR² et où, dans chaque cas, un ou plusieurs atome(s) de H peut/peuvent être remplacé(s) par F, Cl, Br, I, CN ou NO₂, ou un système de cycle aromatique ou hétéroaromatique comportant 5 à 60 atomes de cycle aromatique, lequel peut dans chaque cas être substitué par un radical ou plusieurs radicaux R², ou un groupe aryloxy ou hétéroaryloxy comportant 5 à 60 atomes de cycle aromatique, lequel peut être substitué par un radical ou plusieurs radicaux R², ou une combinaison de ces systèmes ; deux substituants R¹ ou plus peuvent ici également former un système de cycle aliphatique ou aromatique monocyclique ou polycyclique l'un avec l'autre ou les uns avec les autres ;
Ar¹ est, pour chaque occurrence, de manière identique ou différente, un système de cycle aromatique ou hétéroaromatique comportant 5 à 60 atomes de cycle aromatique, lequel peut être substitué par un radical ou plusieurs radicaux R^{2 ;}
R² est, pour chaque occurrence, de manière identique ou différente, H, F ou un radical hydrocarbone aliphatique, aromatique et/ou hétéroaromatique comportant 1 à 20 atome(s) de C, où, en outre, des atomes de H peuvent être remplacés par F ; deux substituants R² ou plus peuvent ici également former un système de cycle aliphatique ou aromatique monocyclique ou polycyclique l'un avec l'autre ou les uns avec les autres ;
Y est, pour chaque occurrence, de manière identique ou différente, un groupe de la formule (2) ou de la formule (3) : 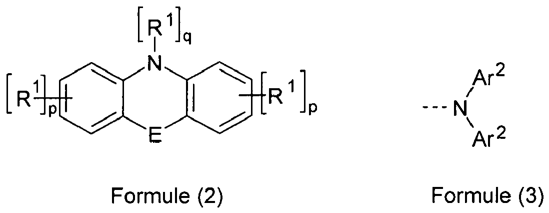 dans lesquelles l'unité of the formule (2) est liée à Ar via n'importe quelle position souhaitée et l'unité de la formule (3) est liée à Ar via N, où R¹ présente la signification indiquée ci avant et en outre :
E représente O, S, N(R¹), P(R¹), P(=O)R¹, C(R¹)₂, Si(R¹)₂ ou une liaison simple ;
Ar² est, de manière identique ou différente pour chaque occurrence, un groupe aryle ou hétéroaryle comportant 5 à 20 atomes de cycle aromatique ou un groupe triarylamine comportant 15 à 30 atomes de cycle aromatique, dont chacun peut être substitué par un radical ou plusieurs radicaux R¹, étant entendu qu'au moins un substituant R¹ qui représente un groupe alkyle ou silyle est présent sur au moins un groupe Ar^{2 ;}
p est, pour chaque occurrence, de manière identique ou différente, 0, 1, 2, 3 ou 4;
q est 0 ou 1, où q = 0 si l'unité de la formule (2) est liée à Ar via l'azote et q = 1 si l'unité de la formule (2) est liée à Ar via un atome autre que l'azote ;
étant entendu que le groupe Ar qui est lié à X et à Y n'est pas conjugué en continu si le composé de la formule (1) présente de façon précise une seule fonction carbonyle ;
à l'exception du composé qui suit :

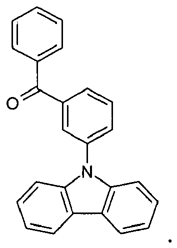

2. Composés des formules (4), (5), (6), (7), (8), (9) et (10) : 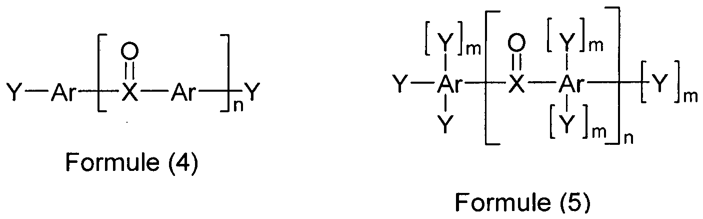 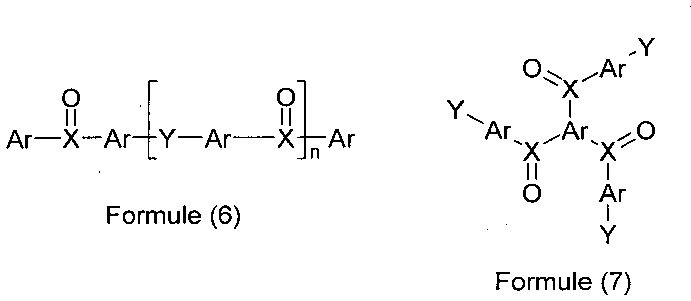 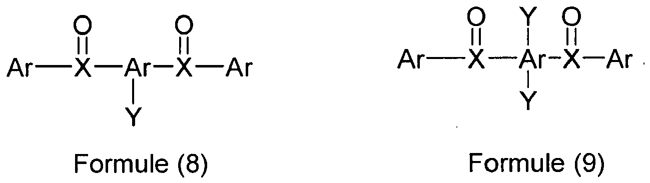 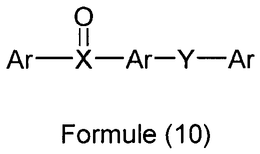 dans lesquelles les symboles et indices présentent les significations indiquées selon la revendication 1, et en outre :
m est, pour chaque occurrence, de manière identique ou différente, 0 ou 1, où m = 0 signifie qu'un groupe R¹ est lié en lieu et place de Y ;
n est, pour chaque occurrence, de manière identique ou différente, 1, 2, 3, 4 ou 5.

3. Composés selon la revendication 1 ou 2, **caractérisés en ce que** le composé contient au moins deux groupes X=O et/ou au moins deux groupes Y.

4. Composés selon une ou plusieurs des revendications 1 à 3, **caractérisés en ce que** le rapport des groupes X=O sur les groupes Y est entre 1 : 10 et 10 : 1.

5. Composés selon une ou plusieurs des revendications 1 à 4, **caractérisés en ce que** le groupe Ar qui est simultanément lié à X et à Y n'est pas conjugué en continu.

6. Composés selon une ou plusieurs des revendications 1 à 5, **caractérisés en ce que** les groupes Ar contiennent seulement des groupes phényle et/ou naphtyle, mais pas des systèmes aromatiques condensés plus grands.

7. Composés selon une ou plusieurs des revendications 1 à 6, **caractérisés en ce que** les groupes Ar qui sont liés à X et à Y sont choisis parmi les unités des formules (11) à (17) : 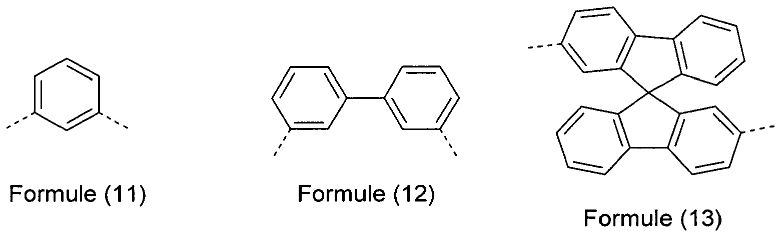 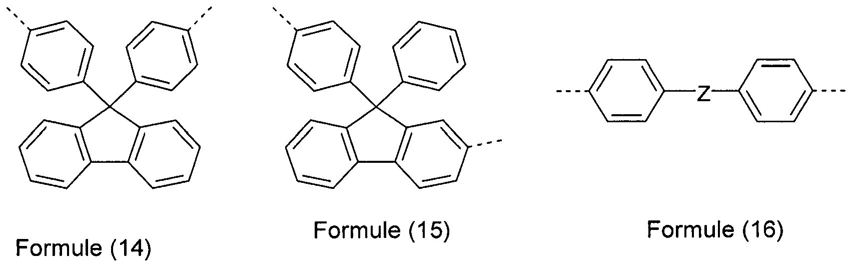 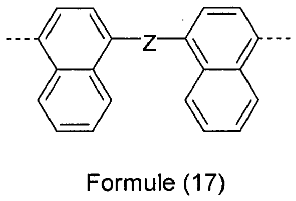 dans lesquelles la liaison en pointillés indique dans chaque cas le lien sur X et sur Y, où les unités peuvent dans chaque cas être substituées par un radical ou plusieurs radicaux R¹ et en outre :
Z est, pour chaque occurrence, de manière identique ou différente, -[C(R¹)₂]ₖ-, Si(R¹)₂, O ou S ;
k est 1, 2, 3, 4, 5 ou 6.

8. Composés selon une ou plusieurs des revendications 1 à 7, **caractérisés en ce que** les unités Y sont, pour chaque occurrence, de manière identique ou différente, un groupe de la formule (2a) ou (3a) : 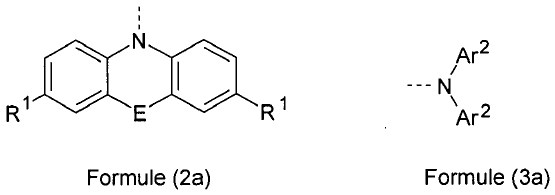 dans lesquelles les unités sont liées à Ar via l'azote, où R¹ présente la signification indiquée ci avant et en outre :
E représente une liaison simple, O, S ou N(R¹) ;
Ar² est, de manière identique ou différente pour chaque occurrence, un groupe aryle ou hétéroaryle comportant 5 à 10 atomes de cycle aromatique ou un groupe triarylamine comportant 18 à 24 atomes de cycle aromatique, dont chacun peut être substitué par un radical ou plusieurs radicaux R¹, étant entendu qu'au moins un substituant R¹ qui représente un groupe alkyle ou silyle est présent sur au moins un groupe Ar².

9. Composés selon une ou plusieurs des revendications 1 à 8, **caractérisés en ce que** le symbole R¹ représente, de manière identique ou différente pour chaque occurrence, H, F, Br, N(Ar¹)₂, P(=O)(Ar¹)₂, C(=O)Ar¹, CR²=CR²Ar¹, un groupe alkyle en chaîne droite comportant 1 à 5 atome(s) de C ou un groupe alkyle ramifié comportant 3 à 5 atomes de C, où un ou plusieurs groupe(s) CH₂ non adjacents peut/ peuvent être remplacé(s) par -R²C=CR²- ou -O- et où un ou plusieurs atome(s) de H peut/peuvent être remplacé(s) par F, ou un groupe aryle comportant 6 à 16 atomes de C ou un groupe hétéroaryle comportant 2 à 16 atomes de C ou un groupe spirobifluorényle, dont chacun peut être substitué par un radical ou plusieurs radicaux R², ou une combinaison de deux ou trois de ces systèmes, ou **en ce que** le symbole R¹ représente également un groupe alkyle en chaîne droite ou ramifié comportant jusqu'à 10 atomes de C si l'unité structurelle de la formule (1) est utilisée dans des polymères ou est traitée à partir d'une solution.

10. Polymères, oligomères ou dendrimères contenant une ou plusieurs unité(s) selon une ou plusieurs des revendications 1 à 9, dans lesquels un radical ou plusieurs radicaux R¹ représente(nt) des liaisons depuis le composé de la formule (1) sur le polymère, l'oligomère ou le dendrimère.

11. Procédé pour la préparation de composés selon une ou plusieurs des revendications 1 à 9, **caractérisé en ce que** le composé est synthétisé par couplage de Hartwig-Buchwald d'un cétone aromatique qui est substitué par un ou plusieurs halogène(s) ou un ou plusieurs groupe(s) OSO₂R² avec un groupe diarylamino, où les deux groupes aryle peuvent également être pontés par un groupe E.

12. Utilisation de composés selon une ou plusieurs des revendications 1 à 10 dans des dispositifs électroniques organiques.

13. Dispositif comprenant une anode, une cathode et au moins une couche organique, **caractérisé en ce qu'**au moins une couche comprend au moins un composé selon une ou plusieurs des revendications 1 à 10, de façon préférable un dispositif électroluminescent organique.

14. Dispositif électroluminescent organique selon la revendication 13, **caractérisé en ce que** le composé selon une ou plusieurs des revendications 1 à 10 est utilisé en tant que matrice pour des dopants phosphorescents dans une couche d'émission.

15. Dispositif électroluminescent organique selon la revendication 14, **caractérisé en ce que** les émetteurs phosphorescents sont choisis parmi des composés des formules (18) à (21) : 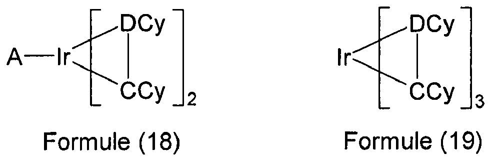 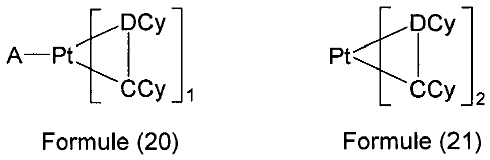 dans lesquelles R¹ présente la même signification comme décrit ci avant et ce qui suit s'applique aux autres symboles utilisés :
DCy est, de manière identique ou différente pour chaque occurrence, un groupe cyclique qui contient au moins un atome de donneur, de façon préférable azote ou phosphore, via lequel le groupe cyclique est lié au métal, et lequel peut à son tour porter un ou plusieurs substituant(s) R^{1 ;} les groupes DCy et CCy sont connectés l'un à l'autre via une liaison covalente ;
CCy est, de manière identique ou différente pour chaque occurrence, un groupe cyclique qui contient un atome de carbone via lequel le groupe cyclique est lié au métal et lequel peut à son tour porter un ou plusieurs substituant(s) R¹;
A est, de manière identique ou différente pour chaque occurrence, un ligand bidenté-chélatant monoanionique, de façon préférable un ligand dicétonate.

16. Dispositif électroluminescent organique selon une ou plusieurs des revendications 13 à 15, **caractérisé en ce que** le composé selon une ou plusieurs des revendications 1 à 10 est introduit dans une intercouche entre une couche d'émission fluorescente et une couche d'émission phosphorescente.
